# EUROPEAN PATENT APPLICATION

(11) **EP 4 831 153 A1**
(43) Date of publication of application: **28.10.2026**
(21) Application number: 25851161.7
(22) Date of filing: 09.07.2025
(51) Int. Cl.: C07C 63/72, B01D 53/047, B01J 20/22, B01J 20/26, B01J 20/34, C07C 65/05, C07C 205/57, C07D 207/34, C07D 401/06, C07F 1/08, C07F 3/06

(54) **METAL COMPLEX, SEPARATING AGENT, AND METHOD FOR SEPARATING TARGET GAS**

(30) Priority: 07.08.2024 JP 2024131234
(71) Applicant: Kyoto University, Kyoto-shi, Kyoto 606-8501 (JP); Resonac Corporation, Tokyo 105-7325 (JP); Crasus Chemical Inc., Oita-shi, Oita 870-0189 (JP)
(72) Inventor: KITAGAWA, Susumu, Kyoto-shi, Kyoto 606-8501 (JP); OTAKE, Ken-ichi, Kyoto-shi, Kyoto 606-8501 (JP); SAKAMOTO, Hirotoshi, Kyoto-shi, Kyoto 606-8501 (JP); WAKISAKA, Takuo, Tokyo 105-7325 (JP); FUJIBE, Satoshi, Oita-shi, Oita 870-0189 (JP); WATANABE, Yoshihiro, Oita-shi, Oita 870-0189 (JP); OTA, Keisuke, Oita-shi, Oita 870-0189 (JP); OKUMURA, Yoshikuni, Tokyo 105-7325 (JP)
(74) Representative: Strehl & Partner mbB
(86) International application number: PCT/JP2025/024673
(87) International publication number: WO 2026/034111

(57) **Abstract**

The purpose of the present invention is to provide a metal complex with which a target gas, especially carbon dioxide, can be separated and recovered from a mixed gas with higher energy efficiency than conventional materials. The metal complex is composed of: a dianion of an aromatic 1,3-dicarboxylic acid compound; a metal dication selected from magnesium, calcium, chromium, molybdenum, tungsten, manganese, iron, ruthenium, cobalt, rhodium, nickel, palladium, copper, zinc and cadmium; and an organic bidentate ligand having a bispyridylethylene structure, and has a structure including two-dimensional sheets stacked in mutual engagement.

## Description

### TECHNICAL FIELD

The content of the present disclosure relates to a metal complex that can be used as a separating agent for selectively adsorbing and desorbing a target gas from a mixed gas containing the target gas, and a method for separating a target gas.

### BACKGROUND ART

In order to achieve carbon neutrality as a countermeasure against global warming, it is desired to develop a separation and recovery technology that can efficiently recover carbon dioxide contained in exhaust gases emitted from various emission sources.

The concentration of carbon dioxide in the exhaust gas greatly varies, depending on the emission source. For example, while exhaust gases containing carbon dioxide at a high concentration of more than 96% may be emitted from manufacturing processes in the chemical industry, the carbon dioxide concentration is approximately less than 30% in exhaust gases emitted at the time of cement or steel manufacture and in combustion exhaust gases emitted at the time of thermal power generation using fossil fuels (NPL 1). Since these exhaust gases are usually released at atmospheric pressure (101 kPaA), the partial pressure of carbon dioxide contained is about 30 kPaA or less.

As separation and recovery methods useful in a case where the concentration of carbon dioxide in the exhaust gas is high, absorption methods such as the hot potassium carbonate process and the amine process are known. These absorption methods, however, require not only a large amount of absorbing material, for example, an amine aqueous solution, but also a large quantity of thermal energy in the step of releasing carbon dioxide absorbed. For this reason, in the case of the exhaust gas having a low carbon dioxide concentration, these absorption methods are inefficient and unsuitable for industrial use.

As a separation and recovery method alternative to the absorption methods, a pressure swing adsorption (PSA) method is known. This is a method utilizing a solid adsorbent (also called a separating agent) that has different adsorption capacities and adsorption rates with respect to two or more kinds of gases. Specifically, an adsorption step in which a mixed gas is supplied to an adsorption tower filled with a separating agent and the pressure is raised to adsorb a specific gas onto the separating agent and a desorption step in which the adsorbed gas is desorbed by reducing the pressure and the gas is discharged from the adsorption tower and recovered are alternately repeated, thereby separating and recovering the specific gas. It has long been known that activated carbon and zeolite are used as separating agents.

These existing separating agents, such as activated carbon and zeolite, have a strong interaction with carbon dioxide, and have a large adsorption amount even in a low partial pressure region of carbon dioxide as well. For this reason, in a case where these separating agents are applied to exhaust gases having a low carbon dioxide partial pressure, that is, a low carbon dioxide concentration, the desorption step has to be carried out at an extremely low pressure. Therefore, a process involving a large pressure fluctuation is required, and the cost required for recovery greatly increases.

The cost for separation and recovery of carbon dioxide by the PSA method using zeolite (13X zeolite) as a separating agent has been estimated by Floudas et al. (NPL 2). Floudas et al. have determined the cost required to recover at least 90% by volume of carbon dioxide in a case where the oxygen concentration is fixed at 5.5% by volume and the carbon dioxide concentration is changed in a range of 1% to 70% by volume in a supplied gas consisting of carbon dioxide, nitrogen, oxygen, and water at 1 bar and 55°C. It is found from this estimation that the cost does not change significantly when the mole fraction of carbon dioxide in the supplied gas is in the range of 0.3 to 0.7, but the cost starts to rise significantly when the mole fraction is less than 0.3.

Meanwhile, the development of porous metal complexes is progressing as porous materials having separation performance. NPLs 3 and 4 report metal complexes in which a dynamic structural change occurs by adsorption and desorption of guest molecules. PTL 1 reports a metal complex, which has a structure in which pseudo-diamond skeletons are triply interpenetrated and is produced from a metal ion, a dicarboxylic acid compound, and a bidentate-coordinating organic ligand, as a metal complex that selectively adsorbs carbon dioxide. PTL 2 reports a metal complex, which has an interpenetrating two-dimensional sheet structure and can be used as an adsorbing material exhibiting carbon dioxide adsorption properties. PTL 3 reports an organometallic complex having a structure in which planes are interdigitated with each other as an organometallic complex for adsorbing and separating carbon dioxide. NPL 5 reports the adsorption behavior of a metal complex having a structure, in which two-dimensional sheets are laminated while being interdigitated with each other, with respect to ethanol and the like.

### CITATION LIST

### PATENT LITERATURE

PTL 1: Japanese Patent Application Publication No. 2013-107826
PTL 2: Japanese Patent Application Publication No. 2013-216622
PTL 3: Japanese Patent No. 4994398

### NON PATENT LITERATURE

NPL 1: Technology Strategy Center Report TSC Foresight Vol. 118 Carbon Recycling Field (CO2 Separation and Recovery Technology) (February 2024), National Research and Development Agency, New Energy and Industrial Technology Development Organization, Technology Strategy Center (TSC)
NPL 2: Ind. Eng. Chem. Res. 2012, 51, 15665-15682
NPL 3: Kazuhiro Uemura, Susumu Kitagawa, Future Materials, Vol. 2, pp. 44-51 (2002)
NPL 4: Ryotaro Matsuda, Susumu Kitagawa, Petrotech, Vol. 26, pp. 97-104 (2003)
NPL 5: Chem. Commun., 2007, 3395-3397

### SUMMARY OF INVENTION

### TECHNICAL PROBLEM

As a method for reducing the separation cost in the PSA method, it is conceivable to narrow the range of pressure fluctuation. From this point of view, the present inventors have focused on metal complexes which exhibit a unique gas adsorption behavior called the gating phenomenon. Such metal complexes are called gate-type metal complexes.

Gate-type metal complexes exhibit dynamic structural changes in response to external stimuli. For example, a gate-type metal complex that exhibits the gating phenomenon in response to the pressure of carbon dioxide has a small adsorption amount of carbon dioxide in a low carbon dioxide partial pressure region, but exhibits a behavior in which the adsorption amount of carbon dioxide rapidly increases when the pressure reaches a certain pressure, that is, the gate opening pressure. The use of a porous metal complex exhibiting such a gating phenomenon as a carbon dioxide separating agent allows the PSA method process to be carried out with a narrow pressure fluctuation range. Hence, the cost of electric power for driving a blower and a pump used for realizing the pressure fluctuation can be reduced, and as a result, the cost of the separation process can be greatly reduced.

However, existing gate-type metal complexes have a problem in that the gate opening pressure is high in an industrially feasible temperature range. In a case where a gate-type metal complex is used as a separating agent, it is required to increase the total pressure of the supplied gas so that the partial pressure of the target gas is equal to or higher than the gate opening pressure and supply the supplied gas to the separating agent. Therefore, in a case where a target gas is recovered from a supplied gas having a low target gas concentration by using a gate-type metal complex having a high gate opening pressure, a large quantity of energy is consumed for raising the pressure of gas components other than the target gas, and this deteriorates the energy consumption rate in the entire process. Hence, it has been difficult to industrially utilize a gate-type metal complex for a supplied gas having a low target gas concentration.

The metal complex of PTL 1 does not exhibit a gating phenomenon with respect to carbon dioxide. Such a metal complex requires an extremely low pressure in the desorption step when carbon dioxide is separated and recovered by the PSA method, and the cost required for recovery increases. PTL 1 reports a metal complex that exhibits a gating phenomenon with respect to ethane, but there has been a problem that the gate opening pressure is as high as 500 to 600 kPaA and the cost for increasing the pressure is high.

PTL 2 reports a metal complex that exhibits a gating phenomenon in adsorption and desorption isotherms of carbon dioxide. However, the gate opening pressure is as high as about 80 kPaA while the gate closing pressure is lower than 20 kPaA, and thus there has been a problem in that it is required to increase the pressure of a supplied gas having a low carbon dioxide concentration and in that the pressure fluctuation range in the PSA method is wide, whereby the cost increases.

PTL 3 indicates that a metal complex produced from zinc nitrate, an isophthalic acid derivative (X=NO₂), and bipyridine exhibits a gating phenomenon in the adsorption isotherm of carbon dioxide at 25°C. However, there has been a problem in that the gate opening pressure is as high as 200 to 300 kPaA and the cost for increasing the pressure is high. PTL 3 also discloses a metal complex in which, in the adsorption isotherm of carbon dioxide, the adsorption isotherm rises sharply from the vicinity of the pressure of 0 kPa and a gating phenomenon is not exhibited. The desorption isotherms of these metal complexes are not disclosed, but generally the desorption isotherms overlap with the adsorption isotherms or shift to lower pressure sides, so it is required to reduce the pressure to the vicinity of 0 kPa in order to separate and recover at least a certain amount of carbon dioxide by desorption. It is thus considered that the energy cost increases.

Hence, the development of a metal complex, which has a low gate opening pressure and can be used to separate and recover a target gas from a mixed gas containing the target gas at a low concentration, has been desired.

The present disclosure provides a metal complex that can be used to separate and recover from a mixed gas a target gas, particularly carbon dioxide, with a higher energy efficiency than conventional materials.

### SOLUTION TO PROBLEM

The present disclosure relates to the following aspects [1] to [21].
[1] A metal complex comprising:
   a dianion of an aromatic 1,3-dicarboxylic acid compound (I);
   a metal dication that is a dication of a metal selected from magnesium, calcium, chromium, molybdenum, tungsten, manganese, iron, ruthenium, cobalt, rhodium, nickel, palladium, copper, zinc, and cadmium; and
   an organic bidentate ligand (II) capable of coordinating to the metal dication, represented by general formula (1) below: 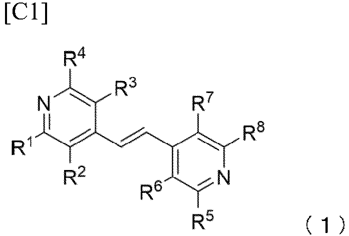 wherein R¹, R², R³, R⁴, R⁵, R⁶, R⁷, and R⁸ are each independently a hydrogen atom, an alkyl group having 1 to 4 carbon atoms, an alkoxy group having 1 to 4 carbon atoms, a formyl group, an acyloxy group, an alkoxycarbonyl group having 2 to 4 carbon atoms, a nitro group, a cyano group, an amino group, a monoalkylamino group having 1 to 4 carbon atoms, a dialkylamino group having 2 to 4 carbon atoms, an acylamino group having 2 to 4 carbon atoms, or a halogen atom,
   the metal complex having a structure in which two-dimensional sheets are laminated while being interdigitated with each other.
[2] The metal complex according to aspect [1], wherein a gate opening pressure and a gate closing pressure are both 1 to 60 kPaA in adsorption and desorption isotherms of carbon dioxide at any measured temperature of 150 to 350 K.
[3] The metal complex according to aspect [1] or [2], wherein the aromatic 1,3-dicarboxylic acid compound (I) is a monocyclic 6-membered aromatic 1,3-dicarboxylic acid compound or a monocyclic 5-membered aromatic 1,3-dicarboxylic acid compound.
[4] The metal complex according to any one of aspects [1] to [3], wherein the aromatic 1,3-dicarboxylic acid compound (I) is a monocyclic 6-membered aromatic 1,3-dicarboxylic acid compound, wherein a ring exhibiting aromaticity in the monocyclic 6-membered aromatic 1,3-dicarboxylic acid compound has at least one substituent selected from a halogen atom, a hydroxy group, and a nitro group in addition to two carboxy groups.
[5] The metal complex according to aspect [4], wherein the aromatic 1,3-dicarboxylic acid compound (I) is represented by general formula (2) below: 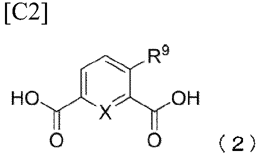 wherein X is CH or N and R⁹ is a halogen atom, a hydroxy group, or a nitro group.
[6] The metal complex according to aspect [1] or [2], wherein the aromatic 1,3-dicarboxylic acid compound (I) is represented by general formula (3) below: 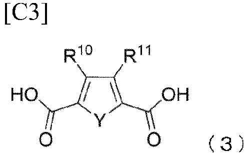 wherein Y is NH, S, or O, R¹⁰ and R¹¹ are each independently a hydrogen atom or an alkoxy group having 1 to 3 carbon atoms, and R¹⁰ and R¹¹ may be bonded to each other to form a ring structure.
[7] The metal complex according to aspect [6], wherein both R¹⁰ and R¹¹ are hydrogen atoms.
[8] The metal complex according to any one of aspects [1] to [7], wherein a composition ratio (molar ratio) of the metal dication, the dianion of the aromatic 1,3-dicarboxylic acid compound (I), and the organic bidentate ligand (II) in the metal complex is 1 : 0.9 to 1.1 : 0.9 to 1.1.
[9] A separating agent comprising the metal complex according to any one of aspects [1] to [8].
[10] A method for separating a target gas, the method comprising:
   bringing a mixed gas comprising a target gas that is to be separated and other gases into contact with the separating agent according to aspect [9], and adsorbing the target gas onto the separating agent; and
   desorbing the target gas.
[11] The method for separating a target gas according to aspect [10], wherein the mixed gas is a gas that comprises at least two kinds selected from an oxygen gas, a nitrogen gas, water vapor, a nitrogen oxide gas, a sulfur oxide gas, a hydrogen gas, a carbon monoxide gas, a carbon dioxide gas, a hydrogen sulfide gas, ammonia, a hydrocarbon gas having 1 to 4 carbon atoms, a noble gas, and siloxane.
[12] The method for separating a target gas according to aspect [10] or [11], wherein the target gas is a carbon dioxide gas.
[13] The method for separating a target gas according to any one of aspects [10] to [12], wherein the mixed gas is a combustion exhaust gas emitted from a and the target gas is a carbon dioxide gas.
[14] A method for separating a target gas, the method comprising:
   bringing a mixed gas comprising a target gas that is to be separated and other gases into contact with a separating agent, and adsorbing the target gas onto the separating agent; and
   desorbing the target gas,
   in which the separating agent comprises a metal complex having a structure in which two-dimensional sheets are laminated while being interdigitated with each other, in which a gate opening pressure and a gate closing pressure are both 1 to 60 kPaA in an adsorption isotherm of carbon dioxide at an operating temperature.
[15] The method for separating a target gas according to aspect [14], wherein the mixed gas is a gas that comprises at least two kinds selected from an oxygen gas, a nitrogen gas, water vapor, a nitrogen oxide gas, a sulfur oxide gas, a hydrogen gas, a carbon monoxide gas, a carbon dioxide gas, a hydrogen sulfide gas, ammonia, a hydrocarbon gas having 1 to 4 carbon atoms, a noble gas, or siloxane.
[16] The method for separating a target gas according to aspect [14] or [15], wherein the target gas is a carbon dioxide gas.
[17] The method for separating a target gas according to any one of aspects [14] to [16], wherein the metal complex is the metal complex according to any one of aspects [1] to [8].
[18] The method for separating a target gas according to aspect [16], wherein a partial pressure of carbon dioxide in the mixed gas is 1 to 30 kPaA.
[19] The method for separating a target gas according to any one of aspects [14] to [18], wherein the operating temperature is 150 to 350 K.
[20] A carbon dioxide separation apparatus comprising a separating agent comprising the metal complex according to any one of aspects [1] to [8].
[21] A metal complex having a structure in which two-dimensional sheets are laminated while being interdigitated with each other, wherein a gate opening pressure and a gate closing pressure are both 1 to 60 kPaA in adsorption and desorption isotherms of carbon dioxide at any measured temperature of 150 to 350 K.

### ADVANTAGEOUS EFFECTS OF INVENTION

According to the present disclosure, it is possible to provide a metal complex that can be used to separate and recover a target gas, particularly carbon dioxide, from a mixed gas with a higher energy efficiency than conventional materials.

### BRIEF DESCRIPTION OF DRAWINGS

[Fig. 1] Fig. 1 is a schematic diagram of a structure (CID structure) in which two-dimensional sheets are laminated while being interdigitated with each other.
[Fig. 2] Fig. 2 is a diagram schematically illustrating adsorption and desorption isotherms for a gate-type metal complex.
[Fig. 3] Fig. 3 is an Oak Ridge Thermal Ellipsoid Plot (ORTEP) diagram of a crystal of a metal complex A obtained in Synthesis Example 1 based on the single crystal X-ray crystal structure analysis.
[Fig. 4] Fig. 4 is a crystal structure model of a metal complex A obtained in Synthesis Example 1.
[Fig. 5-1] Fig. 5-1 is a powder X-ray diffraction pattern of a metal complex A of Synthesis Example 1 before being subjected to vacuum drying.
[Fig. 5-2] Fig. 5-2 is a powder X-ray diffraction pattern of a metal complex A of Synthesis Example 1 after being subjected to vacuum drying.
[Fig. 6] Fig. 6 is an ORTEP diagram of a crystal of a metal complex B obtained in Synthesis Example 2 based on the single crystal X-ray crystal structure analysis.
[Fig. 7] Fig. 7 is a crystal structure model of a metal complex B obtained in Synthesis Example 2.
[Fig. 8] Fig. 8 is a powder X-ray diffraction pattern of a metal complex B of Synthesis Example 2.
[Fig. 9] Fig. 9 is an ORTEP diagram of a crystal of a metal complex C obtained in Synthesis Example 3 based on the single crystal X-ray crystal structure analysis.
[Fig. 10] Fig. 10 is a crystal structure model of a metal complex C obtained in Synthesis Example 3.
[Fig. 11] Fig. 11 is a powder X-ray diffraction pattern of a metal complex D of Synthesis Example 4.
[Fig. 12] Fig. 12 is a powder X-ray diffraction pattern of a metal complex E of Synthesis Example 5.
[Fig. 13] Fig. 13 is a powder X-ray diffraction pattern of a metal complex F of Synthesis Example 6.
[Fig. 14] Fig. 14 is a powder X-ray diffraction pattern of a comparative metal complex P of Comparative Synthesis Example 1.
[Fig. 15] Fig. 15 is a powder X-ray diffraction pattern of a comparative metal complex Q of Comparative Synthesis Example 2.
[Fig. 16] Fig. 16 is an ORTEP diagram of a crystal of a comparative metal complex R obtained in Comparative Synthesis Example 3 based on the single crystal X-ray crystal structure analysis.
[Fig. 17] Fig. 17 is a crystal structure model of a comparative metal complex R obtained in Comparative Synthesis Example 3.
[Fig. 18] Fig. 18 is adsorption and desorption isotherms of carbon dioxide at 298 K for a metal complex A of Synthesis Example 1.
[Fig. 19] Fig. 19 is adsorption and desorption isotherms of carbon dioxide at 195 K for a metal complex B of Synthesis Example 2.
[Fig. 20] Fig. 20 is adsorption and desorption isotherms of carbon dioxide at 195 K for a metal complex C of Synthesis Example 3.
[Fig. 21] Fig. 21 is adsorption and desorption isotherms of carbon dioxide at 298 K for a metal complex D of Synthesis Example 4.
[Fig. 22] Fig. 22 is adsorption and desorption isotherms of carbon dioxide at 298 K for a metal complex E of Synthesis Example 5.
[Fig. 23] Fig. 23 is adsorption and desorption isotherms of carbon dioxide at 298 K for a metal complex F of Synthesis Example 6.
[Fig. 24] Fig. 24 is adsorption and desorption isotherms of carbon dioxide at 298 K for a comparative metal complex Q of Comparative Synthesis Example 2.
[Fig. 25] Fig. 25 is adsorption and desorption isotherms of carbon dioxide at 298 K for a comparative metal complex R of Comparative Synthesis Example 3.
[Fig. 26] Fig. 26 is adsorption and desorption isotherms of carbon dioxide at 195 K for a comparative metal complex S of Comparative Synthesis Example 4.

### DESCRIPTION OF EMBODIMENTS

Hereinafter, the embodiments of the present invention will be described, but it should be understood that the present invention is not limited only to these embodiments, and various applications are possible within the spirit and scope of implementation thereof. In the present disclosure, when "to" is used for the numerical range, the numerical values at both ends are the upper limit value and the lower limit value, respectively, and are included in the numerical range. When a plurality of upper limit values or lower limit values are described, a numerical range can be created from all the combinations of the upper limit values and the lower limit values. Similarly, when a plurality of numerical ranges are described, the upper limit values and the lower limit values can be individually selected from these numerical ranges and combined to create separate numerical ranges.

### [Metal Complex]

The metal complex of an embodiment is composed of a dianion of an aromatic 1,3-dicarboxylic acid compound (I), a specific metal dication, and an organic bidentate ligand (II) capable of coordinating to the metal dication, and has a structure in which two-dimensional sheets are laminated while being interdigitated with each other. The two-dimensional sheet may contain the solvent molecules used at the time of the metal complex production.

### <Aromatic 1,3-dicarboxylic Acid Compound (I)>

In the present disclosure, the aromatic 1,3-dicarboxylic acid compound (I) is a compound in which carboxy groups are each bonded to an atom at the relational positions of the 1- and 3-positions on a ring exhibiting aromaticity. The ring exhibiting aromaticity is preferably a 5-membered ring or 6-membered ring. Here, the relational positions of the 1- and 3-positions refer to substitution types represented by general formulas (4) and (5) for a 5-membered ring and a 6-membered ring, respectively. The same applies to other ring structures. 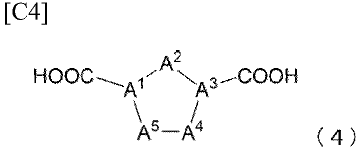
wherein A¹ to A⁵ represent atoms forming a 5-membered ring, which exhibits aromaticity and may have a substituent. 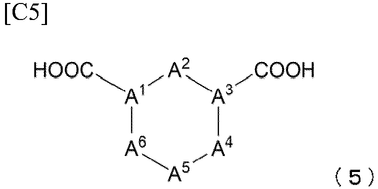
wherein A¹ to A⁶ represent atoms forming a 6-membered ring, which exhibits aromaticity and may have a substituent.

In Formulas (4) and (5), A¹ and A³ are any one of a carbon atom, a nitrogen atom, and a sulfur atom. A² and A⁴ to A⁶ are any one of a carbon atom, a nitrogen atom, an oxygen atom, and a sulfur atom. A hydrogen atom or a substituent may be bonded to the carbon atom or nitrogen atom represented by A² and A⁴ to A⁶. Examples of the substituent include an alkyl group having 1 to 4 carbon atoms, an alkoxy group having 1 to 3 carbon atoms, a halogen atom, a hydroxy group, and a nitro group.

In a case where naming is performed in conformity with the Nomenclature (2013) recommended by the International Union of Pure and Applied Chemistry (IUPAC), when a functional group with a higher priority than a carboxy group is bonded to an aromatic ring, the functional group may be assigned other position numbers (for example, 2,4-positions, 2,5-positions, 3,5-positions, or 2,6-positions) that are not 1,3-positions, but in the present disclosure, these compounds are also regarded as aromatic 1,3-dicarboxylic acid compounds.

Examples of atoms that constitute a ring exhibiting aromaticity include a carbon atom, a nitrogen atom, an oxygen atom, and a sulfur atom. The aromatic 1,3-dicarboxylic acid compound (I) is preferably a monocyclic 6-membered aromatic 1,3-dicarboxylic acid compound or a monocyclic 5-membered aromatic 1,3-dicarboxylic acid compound, more preferably isophthalic acid having a substituent or 2,5-pyrroledicarboxylic acid, which may have a substituent.

### (Monocyclic 6-membered Aromatic 1,3-dicarboxylic Acid Compound)

A monocyclic 6-membered aromatic 1,3-dicarboxylic acid compound refers to an aromatic 1,3-dicarboxylic acid compound in which the number of rings exhibiting aromaticity is one and the ring exhibiting aromaticity is composed of six atoms. The ring exhibiting aromaticity may form a fused ring with a non-aromatic ring. The monocyclic 6-membered aromatic 1,3-dicarboxylic acid compound may have a substituent other than a carboxy group. The ring exhibiting aromaticity in a monocyclic 6-membered aromatic 1,3-dicarboxylic acid compound preferably has at least one substituent selected from a halogen atom, a hydroxy group, and a nitro group in addition to two carboxy groups. Examples of the halogen atom include a fluorine atom, a chlorine atom, a bromine atom, and an iodine atom. The monocyclic 6-membered aromatic 1,3-dicarboxylic acid compound is preferably a compound represented by general formula (2) below. 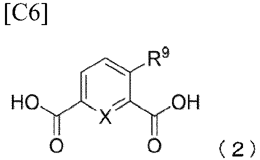 wherein X is CH or N and R⁹ is a halogen atom, a hydroxy group, or a nitro group.

Examples of the halogen atom represented by R⁹ include a fluorine atom, a chlorine atom, a bromine atom, and an iodine atom. The halogen atom is preferably a chlorine atom, a bromine atom, or an iodine atom, and more preferably a bromine atom, from the viewpoint of setting the adsorption pressure and the desorption pressure when the target gas such as carbon dioxide is separated by the PSA method to appropriate ranges.

R⁹ is preferably a bromine atom, a hydroxy group, or a nitro group, and more preferably a hydroxy group from the viewpoint of adsorption and desorption properties as a separating agent.

Specific examples of the monocyclic 6-membered aromatic 1,3-dicarboxylic acid compound include 4-fluoroisophthalic acid, 4-chloroisophthalic acid, 4-bromoisophthalic acid, 4-iodoisophthalic acid, 5-fluoroisophthalic acid, 5-chloroisophthalic acid, 5-bromoisophthalic acid, 5-iodoisophthalic acid, 4-hydroxyisophthalic acid, 5-hydroxyisophthalic acid, 4-nitroisophthalic acid, 5-nitroisophthalic acid, 4-chloro-2,6-pyridinedicarboxylic acid, 4-hydroxy-2,6-pyridinedicarboxylic acid, and 4-nitro-2,6-pyridinedicarboxylic acid. From the viewpoint of reducing the gate opening pressure and gate closing pressure, 4-hydroxyisophthalic acid, 5-bromoisophthalic acid, and 4-nitroisophthalic acid are preferred, and 4-hydroxyisophthalic acid is more preferred.

### (Monocyclic 5-membered Aromatic 1,3-dicarboxylic Acid Compound)

A monocyclic 5-membered aromatic 1,3-dicarboxylic acid compound refers to an aromatic 1,3-dicarboxylic acid compound in which the number of rings exhibiting aromaticity is one and the ring exhibiting aromaticity is composed of five atoms. The ring exhibiting aromaticity may form a fused ring with a non-aromatic ring. The monocyclic 5-membered aromatic 1,3-dicarboxylic acid compound is preferably 2,5-pyrroledicarboxylic acid, which may have a substituent. The monocyclic 5-membered aromatic 1,3-dicarboxylic acid compound is more preferably a compound represented by general formula (3) below. 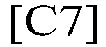 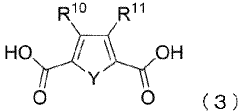 wherein Y is NH, S, or O, R¹⁰ and R¹¹ are each independently a hydrogen atom or an alkoxy group having 1 to 3 carbon atoms, and R¹⁰ and R¹¹ may be bonded to each other to form a ring structure.

From the viewpoint of adsorption and desorption properties as a separating agent, Y is preferably NH or S. In particular, when Y is NH, the gate opening pressure of the metal complex can be reduced.

Examples of the alkoxy group having 1 to 3 carbon atoms represented by R¹⁰ and R¹¹ include a methoxy group, an ethoxy group, a propoxy group, and an isopropoxy group.

R¹⁰ and R¹¹ are preferably hydrogen atoms.

Specific examples of the monocyclic 5-membered aromatic 1,3-dicarboxylic acid compound include 1H-2,5-pyrroledicarboxylic acid, 2,5-thiophenedicarboxylic acid, 3,4-ethylenedioxythiophene-2,5-dicarboxylic acid, and 2,5-furandicarboxylic acid. From the viewpoint of reducing the gate opening pressure and the gate closing pressure, 1H-2,5-pyrroledicarboxylic acid and 3,4-ethylenedioxythiophene-2,5-dicarboxylic acid are preferred, and 1H-2,5-pyrroledicarboxylic acid is more preferred.

A monocyclic 6-membered aromatic 1,3-dicarboxylic acid compound or a monocyclic 5-membered aromatic 1,3-dicarboxylic acid compound and an aromatic 1,3-dicarboxylic acid compound (I) other than these may be used in combination of two or more kinds thereof.

### <Metal Dication>

The metal dication constituting the metal complex is a dication of a metal (also referred to as "metal dication") selected from magnesium, calcium, chromium, molybdenum, tungsten, manganese, iron, ruthenium, cobalt, rhodium, nickel, palladium, copper, zinc and cadmium. Among these, from the viewpoint of reactivity when the metal complex is produced and the availability, a zinc dication (Zn²⁺), a cobalt dication (Co²⁺), and a copper dication (Cu²⁺) are preferred, and a zinc dication and a cobalt dication are more preferred. These metal dications may be used in combination of two or more kinds thereof.

When the metal complex is produced, a salt of the above-mentioned metal can be used as a metal dication source.

### <Organic Bidentate Ligand (II)>

The organic bidentate ligand (II) is a neutral ligand having two moieties that coordinate to a metal dication with unshared electron pairs and is represented by general formula (1) below: 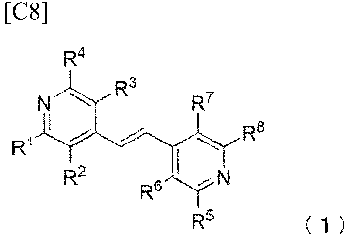 wherein R¹, R², R³, R⁴, R⁵, R⁶, R⁷, and R⁸ are each independently a hydrogen atom, an alkyl group having 1 to 4 carbon atoms, an alkoxy group having 1 to 4 carbon atoms, a formyl group, an acyloxy group, an alkoxycarbonyl group having 2 to 4 carbon atoms, a nitro group, a cyano group, an amino group, a monoalkylamino group having 1 to 4 carbon atoms, a dialkylamino group having 2 to 4 carbon atoms, an acylamino group having 2 to 4 carbon atoms, or a halogen atom.

From the viewpoint of availability, R¹, R², R³, R⁴, R⁵, R⁶, R⁷, and R⁸ are preferably a hydrogen atom or an alkyl group having 1 to 4 carbon atoms, more preferably a hydrogen atom or an alkyl group having 1 to 2 carbon atoms, and particularly preferably all a hydrogen atom. When R¹, R², R³, R⁴, R⁵, R⁶, R⁷, and R⁸ are all a hydrogen atom, the organic bidentate ligand (II) is trans-1,2-bis(4-pyridyl)ethylene.

The organic bidentate ligand (II) may be used in combination of two or more kinds thereof.

### <Structure of Metal Complex>

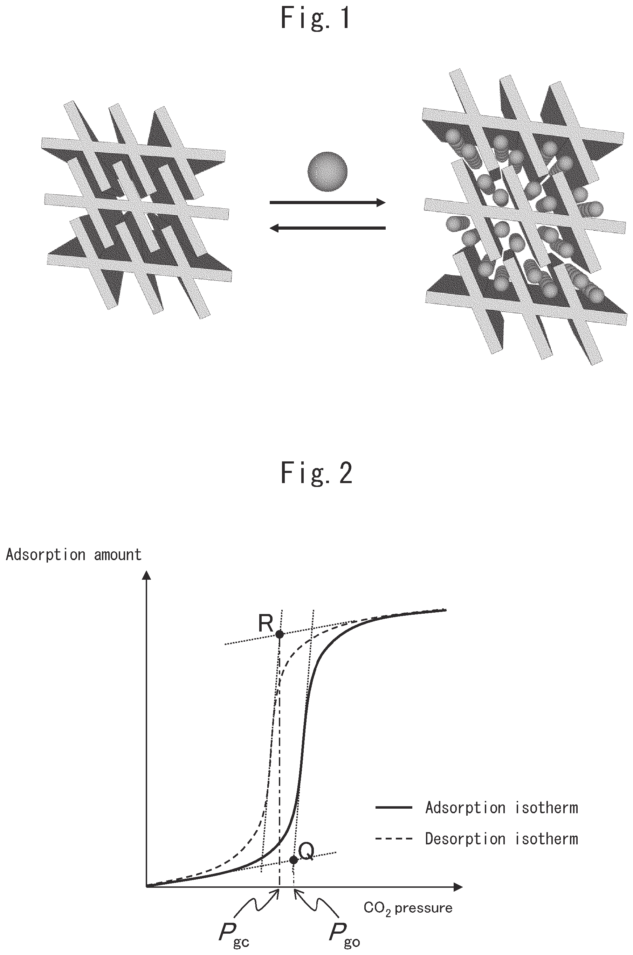

The metal complex has a structure in which two-dimensional sheets constructed by a covalent bond and a coordinate bond are laminated while being interdigitated with each other. The term "interdigitation with each other" means a structure in which two-dimensional sheets interlock with each other, and that there is no covalent bond or coordinate bond between the sheets. Since there is no covalent bond or coordinate bond between the two-dimensional sheets, when the two-dimensional sheets come into contact with a gas having a pressure (partial pressure in the case of a mixed gas) equal to or higher than a certain pressure, the sheets are displaced to expand the space between the sheets, so that the gas molecules can be trapped and adsorbed between the sheets. Fig. 1 is a schematic diagram illustrating the situation in which gas molecules represented by spheres are adsorbed and desorbed onto a structure in which two-dimensional sheets are laminated while being interdigitated with each other.

In the metal complex, a two-dimensional sheet is constituted by the coordinate bond of a dianion of an aromatic 1,3-dicarboxylic acid compound (I) and an organic bidentate ligand (II) to a metal dication. This two-dimensional sheet has a framework structure in which projections like fins are projected, and the fins are engaged with each other to construct a structure in which two-dimensional sheets are laminated while being interdigitated with each other. Such a structure is also called the Coordination polymer with Interdigitated Structure (CID structure) (NPL 5: Chem. Commun., 2007, 3395-3397). More specifically, the metal complex is a structural body in which the organic bidentate ligand (II) coordinates to the metal dication in a planar manner to form a planar, that is, two-dimensional sheet, the dianion of the aromatic 1,3-dicarboxylic acid compound (I) coordinates to the metal dication from the axial direction to form a fin portion, a plurality of two-dimensional sheets, each composed of the organic bidentate ligand (II) and the metal dication, are laminated, and these two-dimensional sheets are interdigitated with each other by the dianion portion of the aromatic 1,3-dicarboxylic acid compound (I).

The fact that the metal complex has a structure in which two-dimensional sheets are laminated while being interdigitated with each other can be confirmed by, for example, single crystal X-ray structure analysis and powder X-ray analysis. Specifically, the fact can be confirmed by the method described in Examples. When a metal complex is produced using the same constituent materials as well, a structure in which two-dimensional sheets are laminated while being interdigitated with each other may not be constructed if the production method is different, and the metal complex according to the present disclosure can be distinguished from another metal complex by an analysis method such as single crystal X-ray structure analysis or powder X-ray analysis.

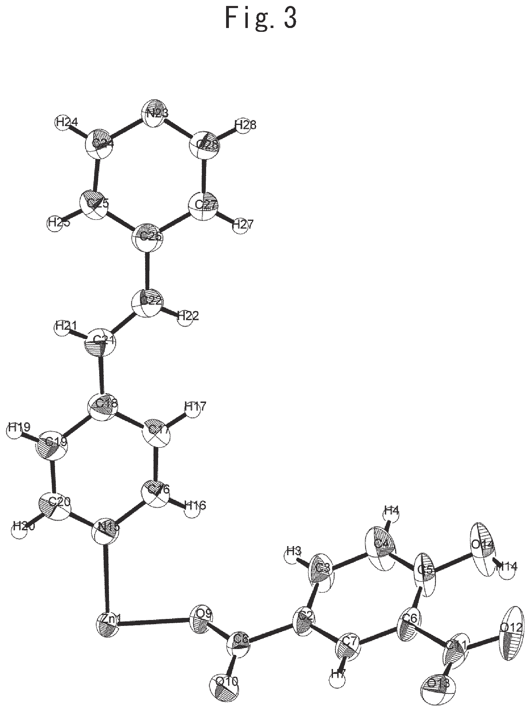

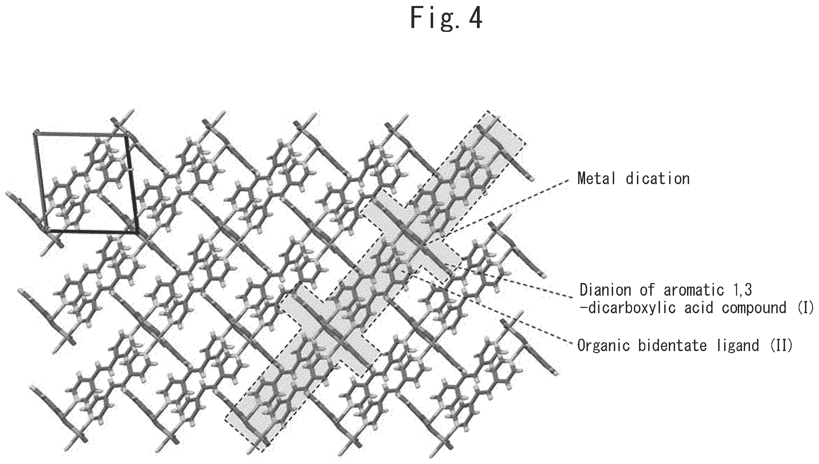

By carrying out single crystal X-ray structure analysis, as presented in Table 1, for example, the crystal system, space group, lattice constant and the like can be determined for the crystal of a metal complex. Furthermore, since the atomic coordinates are also determined, the structure of the metal complex can be three-dimensionally drawn using a specific drawing program, for example, as an Oak Ridge Thermal Ellipsoid Plot (ORTEP) diagram illustrated in Fig. 3, a wire frame diagram, a capped sticks diagram or the like. The fact that the metal complex has a structure in which two-dimensional sheets are laminated while being interdigitated with each other can be confirmed by drawing a crystal structure model in which unit lattices are three-dimensionally arranged as illustrated in Fig. 4. In Fig. 4, the structure in which the two-dimensional sheets are interdigitated with each other is read, by the two-dimensional sheet consisting of an organic bidentate ligand (II) and a metal dication and a dianion of an aromatic 1,3-dicarboxylic acid compound (I) standing perpendicular to the sheet.

The diffraction pattern acquired by the powder X-ray analysis depends on the arrangement of atoms constituting the crystal, and is thus intrinsic to the crystal structure. For a metal complex, which has been found to have a structure in which two-dimensional sheets are laminated while being interdigitated with each other by the single crystal X-ray structure analysis, a powder X-ray diffraction pattern yields a powder X-ray diffraction pattern peculiar to this structure. By comparing this peculiar powder X-ray diffraction pattern with the powder X-ray diffraction pattern of a given metal complex, it is possible to determine whether the metal complex has a structure in which two-dimensional sheets are laminated while being interdigitated with each other.

The composition ratio (molar ratio) of the metal dication, the dianion of the aromatic 1,3-dicarboxylic acid compound (I), and the organic bidentate ligand (II) in the metal complex is preferably 1 : 0.9 to 1.1 : 0.9 to 1.1. From the viewpoint of high purity of the structure in which two-dimensional sheets are laminated while being interdigitated with each other and high adsorption performance, the composition ratio (molar ratio) is more preferably 1 : 0.95 to 1.05 : 0.95 to 1.05, and particularly preferably 1 : 0.98 to 1.02 : 0.98 to 1.02.

### <Method for Producing Metal Complex>

The metal complex can be produced, for example, by reacting an aromatic 1,3-dicarboxylic acid compound (I) with a salt of a metal selected from magnesium, calcium, chromium, molybdenum, tungsten, manganese, iron, ruthenium, cobalt, rhodium, nickel, palladium, copper, zinc, and cadmium and an organic bidentate ligand (II) capable of coordinating to the dication of the metal in a solvent under normal pressure for several hours to several days, and then performing precipitation.

Examples of the salt of a metal include organic acid salts such as an acetate and a formate and inorganic acid salts such as a chloride, a bromide, a sulfate, a nitrate, and a carbonate. It is preferred to use a single metal salt as the salt of a metal, but two or more kinds of metal salts may be used in mixture.

The mixing ratio (molar ratio) of the organic bidentate ligand (II) to the aromatic 1,3-dicarboxylic acid compound (I) when the metal complex is produced is such that organic bidentate ligand (II) : aromatic 1,3-dicarboxylic acid compound (I) is preferably in the range of 1 : 8 to 1 : 0.2 and more preferably in the range of 1 : 6 to 1 : 0.33. When the reaction is conducted in a range other than these as well, the desired metal complex can be obtained but side reactions increase and the yield decreases in some cases.

The mixing ratio (molar ratio) of the organic bidentate ligand (II) to the salt of a metal when the metal complex is produced is such that organic bidentate ligand (II) : salt of metal is preferably in the range of 1 : 3 to 1 : 0.33, and more preferably in the range of 1 : 2 to 1 : 0.5. In a range other than these, the yield of the desired metal complex may decrease or unreacted raw materials may remain and purification of the obtained metal complex may be difficult.

The molar concentration of the aromatic 1,3-dicarboxylic acid compound (I) in the solution for producing the metal complex is preferably 0.005 to 5.0 mol/L, and more preferably 0.01 to 2.0 mol/L. Although the desired metal complex can be obtained, it is not preferred to conduct the reaction at a concentration lower than this since the yield decreases. The solubility decreases at a concentration higher than this, and the reaction does not proceed smoothly in some cases.

The molar concentration of the salt of a metal in the solution for producing the metal complex is preferably 0.005 to 5.0 mol/L, and more preferably 0.01 to 2.0 mol/L. Although the desired metal complex can be obtained, it is not preferred to conduct the reaction at a concentration lower than this since the yield decreases. At a concentration higher than this, unreacted salts of the metals may remain and purification of the obtained metal complex may be difficult.

The molar concentration of the organic bidentate ligand (II) in the solution for producing the metal complex is preferably 0.005 to 5.0 mol/L, and more preferably 0.01 to 2.0 mol/L. Although the desired metal complex can be obtained, it is not preferred to conduct the reaction at a concentration lower than this since the yield decreases. The solubility decreases at a concentration higher than this, and the reaction does not proceed smoothly in some cases.

It is considered that production of the metal complex proceeds as the metal dication, the dianion of the aromatic 1,3-dicarboxylic acid compound (I), and the organic bidentate ligand (II), solvated in a solution, precipitate as crystals from the solution while forming a structure in which two-dimensional sheets are laminated while being interdigitated with each other by a reversible coordinate bond. When a structure in which two-dimensional sheets are laminated while being interdigitated with each other is formed, it is considered that the formation reaction of the metal complex proceeds while encapsulating solvent molecules, particularly solvent molecules such as N,N-dimethylformamide, N,N-dimethylacetamide, using these solvent molecules as a template. In other words, it is considered that the structure of the metal complex is a structure in which the sum of energy based on the interactions respectively working between the solvent molecules acting as a template and the components constituting the metal complex, that is, the metal dication, the dianion of the aromatic 1,3-dicarboxylic acid compound (I), and the organic bidentate ligand (II), which are contained in the solvent used, is minimized. Therefore, the structure of the metal complex can be adjusted by selecting an appropriate kind of solvent and the volume mixing ratio, depending on the combination of raw materials.

Examples of the solvent used in the production of the metal complex include an organic solvent, water, and a mixed solvent thereof. Specific examples include methanol, ethanol, n-propanol, isopropanol, diethyl ether, dimethoxyethane, tetrahydrofuran, hexane, cyclohexane, heptane, benzene, toluene, methylene chloride, chloroform, acetone, ethyl acetate, acetonitrile, N,N-dimethylformamide (DMF), N,N-dimethylacetamide (DMAc), water, and mixed solvents thereof. Among these, from the viewpoint of efficiently obtaining a metal complex having a structure in which two-dimensional sheets are laminated while being interdigitated with each other, a solvent comprising at least one of N,N-dimethylformamide and N,N-dimethylacetamide, for example, N,N-dimethylformamide or N,N-dimethylacetamide alone, or a mixed solvent of N,N-dimethylformamide or N,N-dimethylacetamide with water is preferred. Particularly preferred solvents are N,N-dimethylformamide alone, and a mixed solvent of N,N-dimethylformamide with water. The volume mixing ratio of N,N-dimethylformamide to water is preferably water/N,N-dimethylformamide (volume ratio before mixing) = 0 to 1.5, and particularly preferably 0 to 1.0.

The solvent molecules, which have acted as a template at the time of synthesis and contributed to the formation of the structure of the metal complex, can be removed from the metal complex by subsequent operations such as normal pressure drying and vacuum drying. The solvent molecules are preferably removed from the viewpoint of forming a space in which molecules of the target gas to be separated are adsorbed when the metal complex is used as a gas separating agent. However, the solvent may remain as long as the function of the metal complex as a separating agent is not impaired. The amount of the remaining solvent is preferably 1% by mass or less in the metal complex. The amount of the remaining solvent is, for example, 0.001% by mass or more in the metal complex.

The reaction temperature is not particularly limited, and is preferably -20°C to 200°C, more preferably 10°C to 150°C, and particularly preferably 50°C to 120°C. It is disadvantageous that the reaction temperature is too low from the viewpoint of the production cost since the reaction time increases. It is disadvantageous that the reaction temperature is too high from the viewpoint of the production cost since the cost required for heating increases.

The reaction time is not particularly limited, and is preferably 72 hours or less, and more preferably 48 hours or less. It is disadvantageous that the reaction time is longer than this from the viewpoint of production cost.

The atmosphere at the time of synthesis is not particularly limited, and an inert gas atmosphere is preferred from the viewpoint of increasing the yield. Examples of the inert gas include nitrogen gas and argon gas. In an air atmosphere, the aromatic 1,3-dicarboxylic acid compound (I) and the organic bidentate ligand (II) may deteriorate, for example, due to oxidation, resulting in a reduced yield.

The reaction pressure is not particularly limited, and is preferably 0.01 to 5 MPaA, more preferably 0.05 to 3 MPaA, and particularly preferably 0.1 to 0.5 MPaA.

A metal complex exhibiting favorable crystallinity has a high purity and favorable adsorption performance. In order to enhance the crystallinity, at the start of the reaction, a suitable acid or base is preferably added, in addition to the salt of a metal, the aromatic 1,3-dicarboxylic acid compound (I), and the organic bidentate ligand (II), by 0.1 to 100 equivalents with respect to the organic bidentate ligand (II) in order to adjust the rate of the metal complex precipitating from the reaction solvent.

As the reaction proceeds and a metal complex is formed, the metal complex precipitates as a solid from the reaction solvent. The completion of the reaction can be confirmed by determining the remaining amount of the raw materials by a known analysis method such as gas chromatography or high performance liquid chromatography. After completion of the reaction, for example, the obtained mixed solution is subjected to suction filtration to separate the precipitated metal complex, and the separated metal complex is washed with an organic solvent to obtain a metal complex.

The crystals of the metal complex obtained may be further dried. Examples of the drying operation include vacuum drying and normal pressure drying. In a case where adsorbed water, solvent molecules, and the like are contained in the crystals of the metal complex, these can be removed by the operation. The drying temperature is preferably 40°C to 250°C, more preferably 45°C to 230°C, and still more preferably 50°C to 220°C. It is disadvantageous that the temperature is too low from the viewpoint of production cost since drying takes time. When the temperature is too high, the metal complex is liable to be decomposed. In the case of vacuum drying, the pressure for vacuum drying is preferably 100 Pa or less, and more preferably 50 Pa or less. It is disadvantageous that the pressure is too high from the viewpoint of production cost since drying takes time. The drying time is preferably 72 hours or less, and more preferably 24 hours or less.

### [Separating Agent]

The metal complex of an embodiment can be used as a separating agent for separating a target gas from a mixed gas containing the target gas to be separated and other gases.

### <Gate-type Metal Complex>

The metal complex of an embodiment is a metal complex that exhibits gate-type adsorption and desorption isotherms with respect to a target gas such as carbon dioxide (CO₂), that is, a gate-type metal complex. The metal complex of an embodiment is a metal complex in which both the gate opening pressure and the gate closing pressure are 1 to 60 kPaA in the adsorption and desorption isotherms of carbon dioxide at any measured temperature of 150 to 350 K and which has a structure in which two-dimensional sheets are laminated while being interdigitated with each other. Fig. 2 is a diagram illustrating gate-type CO₂ adsorption and desorption isotherms, where a solid line represents an adsorption isotherm and a dashed line represents a desorption isotherm. The "gating phenomenon" is a phenomenon in which the adsorption isotherm does not show adsorption at all or rises gently in the region where the pressure (partial pressure in the case of a mixed gas) of the gas to be adsorbed and desorbed, that is, the target gas is low, while the adsorption isotherm sharply rises when the pressure approaches the gate opening pressure P_{go} to be described later and then the slope again disappears or moderates when the pressure passes the gate opening pressure. In the case of the desorption isotherm, the route is reversed. In a case where the adsorption isotherm sharply rises from the region where the pressure of the target gas is low, that is, in the vicinity of 0 kPa and then only shifts to an approximately horizontal portion, such behavior is not referred to as a gating phenomenon in the present disclosure. In such a case, it is required to considerably decrease the pressure when a certain amount or more of the target gas is desorbed and recovered, and thus the energy cost increases in some cases. In a case where the metal complex exhibits the gating phenomenon, in the desorption step, it is sufficient to reduce the pressure to a pressure slightly lower than the gate closing pressure P_{gc} to be described later, and thus the energy cost and the working time of the apparatus can be suppressed.

In gate-type adsorption isotherms, the adsorption amount of the gas remains small until the pressure reaches a specific pressure P_{go}, but when the pressure reaches the pressure P_{go}, the structure of the metal complex changes, for example, by the widening of the space between the laminated two-dimensional sheets, and the metal complex adsorbs the gas rapidly. In the present disclosure, this P_{go} is called the "gate opening pressure". Conversely, in a case where the pressure is decreased as well, when the pressure is lower than P_{gc}, the structure of the metal complex changes, for example, the distance between the laminated two-dimensional sheets is narrowed, and the metal complex desorbs the gas rapidly. In the present disclosure, this P_{gc} is called the "gate closing pressure". Such a behavior is considered to be because, at a low pressure, it is more energetically stable for the metal complex and gas molecules to exist separately, while, once gate opening pressure is reached, incorporation of the gas molecules into the metal complex forms a more stable inclusion complex and becomes more energetically stable. In a case where the pressure shifts from a high pressure to a low pressure, the opposite phenomenon occurs once the pressure falls below the gate closing pressure.

In the present disclosure, the gate opening pressure P_{go} is the intersection of a tangential line at the inflection point in a region where the adsorption amount rapidly increases on the adsorption isotherm and an extended line of a portion, which is located on the lower pressure side than the inflection point and has a constant slope. In Fig. 2, the pressure at the intersection Q is the gate opening pressure P_{go}. In a case where two or more portions having a constant slope are observed, an extended line of a portion, which is located on the highest pressure side and has a constant slope, is used. The gate closing pressure P_{gc} is the intersection of a tangential line at the inflection point in a region where the adsorption amount rapidly decreases on the desorption isotherm and an extended line of a portion, which is located on the higher pressure side than the inflection point and has a constant slope. In Fig. 2, the pressure at the intersection R is the gate closing pressure P_{gc}. In a case where two or more portions having a constant slope are observed, an extended line of a portion, which is located on the lowest pressure side and has a constant slope, is used. In the present disclosure, the adsorption and desorption isotherms are measured by the method described in Examples.

The gate opening pressure and the gate closing pressure vary, depending on the temperature, and generally the gate opening pressure and the gate closing pressure change to a low pressure when the temperature is set to a low temperature, and the gate opening pressure and the gate closing pressure change to a high pressure when the temperature is set to a high temperature. In the gate-type metal complex of an embodiment, both the gate opening pressure and the gate closing pressure are 1 to 60 kPaA in the adsorption and desorption isotherms of carbon dioxide at any measured temperature of 150 to 350 K. The gate opening pressure and gate closing pressure may be 1 kPaA or more, 2 kPaA or more, or 3 kPaA or more, and may be 60 kPaA or less, 50 kPaA or less, or 30 kPaA or less. From the viewpoint of improving the energy efficiency, the gate opening pressure and the gate closing pressure are more preferably 2 to 50 kPaA, and particularly preferably 3 to 30 kPaA. The term "any measured temperature" means at least one measured temperature in a range of 150 to 350 K. In other words, the gate-type metal complex of an embodiment exhibits a gate opening pressure and a gate closing pressure of 1 to 60 kPaA, 2 to 50 kPaA, or 3 to 30 kPaA in the entire range, at a part, or at one point of the temperature range of 150 to 350 K. The adsorption isotherm and desorption isotherm for determining the gate opening pressure and the gate closing pressure are measured at the same temperature. From the viewpoint of cost reduction, the gate-type metal complex has both a gate opening pressure and a gate closing pressure of preferably 1 to 60 kPaA, more preferably 2 to 50 kPaA, and still more preferably 3 to 30 kPaA in the adsorption and desorption isotherms of carbon dioxide at 298 K.

### <Shaping into Separating Agent>

The separating agent of an embodiment contains a metal complex. The separating agent of an embodiment is a shaped body of a metal complex. The separating agent may contain additives such as a lubricant and a binder. The separating agent can be used, for example, as an irregular filling material shaped into beads, rings, strands, or tablets, or as a regular structural body, for example, a regular filling material, a honeycomb body, or a monolith. As a method for shaping the metal complex, known methods such as tableting can be used. The separating agent is usually used by being filled in a container such as an adsorption tower or a tank.

### [Method for Separating Target Gas]

The separating agent can selectively adsorb and separate a target gas in a mixed gas containing the target gas to be separated and other gases. In other words, by bringing the mixed gas into contact with the separating agent to adsorb the target gas onto the separating agent, and then desorbing the target gas, the target gas can be separated. The adsorption and desorption operation is preferably carried out by a pressure swing adsorption (PSA) method. In an embodiment, the separating agent can selectively adsorb and separate only a target gas in a mixed gas containing the target gas to be separated and other gases.

### <Mixed Gas and Target Gas>

The mixed gas is a gas that contains at least two kinds selected from oxygen gas, nitrogen gas, water vapor, a nitrogen oxide gas, a sulfur oxide gas, hydrogen gas, carbon monoxide gas, carbon dioxide gas, hydrogen sulfide gas, ammonia, a hydrocarbon gas having 1 to 4 carbon atoms, a noble gas, and a siloxane. Examples of the hydrocarbon gas having 1 to 4 carbon atoms include methane, ethane, ethylene, and acetylene. Examples of the noble gas include helium, neon, argon, krypton, and xenon. Examples of the siloxane include hexamethylcyclotrisiloxane and octamethylcyclotetrasiloxane. The mixed gas may contain other organic vapors and the like.

The organic vapor refers to a vaporized gas of an organic substance that is liquid at normal temperature and normal pressure. Examples of such an organic substance include alcohols such as methanol and ethanol; amines such as trimethylamine; aldehydes such as acetaldehyde; aliphatic hydrocarbons having 5 to 16 carbon atoms; aromatic hydrocarbons such as benzene and toluene; ketones such as acetone and methyl ethyl ketone; and halogenated hydrocarbons such as methyl chloride and chloroform.

Preferred examples of the gases contained in the mixed gas include oxygen gas, nitrogen gas, carbon dioxide gas, water vapor, a nitrogen oxide gas, and a sulfur oxide gas, and more preferred examples of the gases include oxygen gas, nitrogen gas, carbon dioxide gas, and water vapor, and still more preferred examples of the gases include oxygen gas, nitrogen gas, and carbon dioxide gas.

The target gas is not particularly limited as long as it is a gas contained in the mixed gas, and the target gas may be one kind or two or more kinds. The target gas is preferably carbon dioxide gas.

Since the metal complex of an embodiment exhibits a low gate opening pressure with respect to carbon dioxide, the metal complex can efficiently separate and recover carbon dioxide from a mixed gas containing carbon dioxide at a low concentration. The carbon dioxide separation apparatus of an embodiment comprises a separating agent containing a metal complex.

In one preferred aspect of the present disclosure, the mixed gas containing carbon dioxide gas is combustion exhaust gas emitted from various plants. Specific examples include exhaust gas from a naphtha cracker, exhaust gas from natural gas-thermal power generation, exhaust gas from coal-thermal power generation, exhaust gas from an iron-manufacturing plant, and exhaust gas from a cement plant.

The partial pressure of carbon dioxide in the mixed gas containing carbon dioxide gas may be 1 kPaA or more, 2 kPaA or more, or 3 kPaA or more, and may be 30 kPaA or less, 25 kPaA or less, or 20 kPaA or less. The partial pressure of carbon dioxide in the mixed gas is preferably 1 to 30 kPaA, more preferably 2 to 25 kPaA, and particularly preferably 3 to 20 kPaA. In order to efficiently adsorb carbon dioxide onto the separating agent, it is preferable to set the partial pressure of carbon dioxide to be equal to or higher than the gate opening pressure. Therefore, when the partial pressure of carbon dioxide is 1 kPaA or more, the quantity of the increase in pressure of the mixed gas can be diminished, and the energy efficiency for the separation process is improved. On the other hand, in order to efficiently desorb carbon dioxide, it is preferable to set the partial pressure of carbon dioxide to be equal to or lower than the gate closing pressure. Therefore, when the partial pressure of carbon dioxide is 30 kPaA or less, the quantity of the decrease in pressure can be diminished, and the energy efficiency for the separation process is improved.

### <Pressure Swing Adsorption (PSA) Method>

The separating agent can be used as a separating agent to be filled in the adsorption tower in a pressure swing adsorption (PSA) method. The size of the adsorption tower and the amount of separating agent to be filled can be adjusted, depending on the amount of mixed gas to be treated, the partial pressure of the target gas in the mixed gas, and the like.

The apparatus to be used in the PSA method is not particularly limited, and methods described in known literatures (for example, Newest Adsorption Technology Handbook, Newly-Revised Third Edition NTS Publishing 2020, pp.. 141-145) can be used.

The adsorption tower may be equipped with the equipment for increasing or decreasing the temperature of the filled separating agent. The equipment is not particularly limited, and examples thereof include equipment having a heating device or a cooling device externally installed, and equipment having a heating device or a cooling device internally installed.

The operating temperature may be 150 K or more, 190 K or more, or 280 K or more, and may be 350 K or less, 340 K or less, or 320 K or less. The operating temperature is preferably 150 to 350 K, more preferably 190 to 340 K, and particularly preferably 280 to 320 K. When the operating temperature is 150 K or more, the cost required for cooling can be reduced. When the operating temperature is 320 K or less, the decrease in the adsorption amount can be suppressed. In the present disclosure, the operating temperature refers to the temperature of the separating agent when adsorption and desorption are performed.

In the PSA method, a plurality of adsorption towers are usually used. The number of adsorption towers is not particularly limited, and in the case of using a plurality of adsorption towers, the separating agent of an embodiment can be filled in at least one adsorption tower, preferably all of the adsorption towers.

### EXAMPLES

Hereinafter, the present invention will be more specifically described with reference to Examples, but the present invention is not limited to these Examples. Analyses and evaluations in Examples and Comparative Examples were conducted as follows.

### (1) Single Crystal X-ray Structure Analysis

The measurement was carried out using a single crystal X-ray diffractometer XtaLAB P200 (Rigaku Corporation) under cooling by nitrogen flow at 195 K. As X-rays, Kα rays of Mo (λ = 0.71075 Å) were used. The structure was solved by direct method (SHELXT) and refined by the total matrix least squares method (SHELXL). Atoms other than hydrogen were anisotropically optimized and hydrogen atoms were positioned using AFIX Instruction.

### (2) Measurement of Powder X-ray Diffraction Pattern

X-ray diffractometers: Miniflex600-C, SmartLab, or Multiflex (all manufactured by Rigaku Corporation) were used, scanning was performed in a diffraction angle (2θ) range of 3° to 40° at a scanning speed of 5°/min, and the measurement was carried out by the symmetric reflection method.

### (3) Measurement of Adsorption and Desorption Isotherms

The measurement was carried out by the capacitance method (equilibrium waiting time: 300 seconds or more) using a gas adsorption amount measuring apparatus (MicrotracBEL Corp., BELSORP MAX or BELSORP MAX II). The measured temperature was set to 195 K or 298 K and the lower limit of the measuring pressure range was set to 0 kPaA and the upper limit thereof was set to 100 kPaA. Prior to the measurement, the sample was dried at a temperature of 130°C or more and a pressure of 50 Pa or less for 2 hours or more using a pretreatment apparatus (MicrotracBEL Corp., BELPREP VAC II) to remove solvent molecules, adsorbed water and the like.

### <Synthesis Example 1> Synthesis of Metal Complex A

In 120 mL of N,N-dimethylformamide and 100 mL of water, 1.19 g (4.0 mmol) of zinc nitrate hexahydrate, 0.729 g (4.0 mmol) of 4-hydroxyisophthalic acid, and 0.729 g (4.0 mmol) of trans-1,2-bis(4-pyridyl)ethylene were dissolved, and the mixture was stirred at normal pressure and 95°C for 16 hours. A part of the precipitated crystals was taken out and subjected to single crystal X-ray structure analysis. The ORTEP diagram acquired by the single crystal X-ray structure analysis is illustrated in Fig. 3. The crystals contained N,N-dimethylformamide, which was a solvent molecule, but these solvent molecules were omitted. As a result of the structure analysis, it was found that the obtained metal complex had the composition of zinc dication : dianion of 4-hydroxyisophthalic acid : trans-1,2-bis(4-pyridyl)ethylene = 1 : 1 : 1. Table 1 presents the lattice constant and the like.

By the single crystal X-ray structure analysis, the arrangement of the metal dication, the dianion of aromatic 1,3-dicarboxylic acid compound (I), and the organic bidentate ligand (II) in the crystal, that is, the crystal structure is determined. Fig. 4 illustrates the crystal structure model of the metal complex A. The region surrounded by a dashed line represents one of the two-dimensional sheets in which projections like fins are projected. It can be seen from Fig. 4 that the organic bidentate ligand (II) is coordinated in the planar direction of the metal dication to form a two-dimensional sheet, and the dianion of the aromatic 1,3-dicarboxylic acid compound (I) coordinated to the metal dication projects in a direction approximately perpendicular to the two-dimensional sheet to form a fin-like structure. Furthermore, the two-dimensional sheets are laminated while these fin-like structures are interdigitated with each other, and from this fact, it can be seen that the crystal of the metal complex A has a structure (CID structure) in which two-dimensional sheets are laminated while being interdigitated with each other.

### [Table 1]

**Table 1**

| Number of Synthesis Example | | | Synthesis Example 1 | Synthesis Example 2 | Synthesis Example 3 (*) | Comparative Synthesis Example 3 |
|---|---|---|---|---|---|---|
| | | | Metal complex A | Metal complex B | Metal complex C | Comparative metal complex R |
| Molecular formula | | | C₂₀H₁₄N₂O₅Zn | C₄₀H₂₆Br₂N₄O₈Zn₂ | C₆₉H₆₀N₁₂O₂₁Zn₃ | C₂₀H₁₃N₃O₆Zn |
| Molecular weight | | [g/mol] | 427.7 | 981.2 | 1589.4 | 456.7 |
| Crystal system | | | Triclinic | Monoclinic | Triclinic | Monoclinic |
| Space group | | | *P*-1 | *P* 2₁/*n* | *P*-1 | *P* 2₁/*n* |
| Lattice constant | *a* | [Å] | 9.3797(10) | 10.0798(3) | 9.9768(3) | 7.6070(4) |
| | *b* | [Å] | 9.9429(9) | 13.7076(8) | 11.4355(5) | 17.8013(7) |
| | c | [Å] | 11.0424(10) | 32.6213(12) | 30.5989(14) | 14.3379(7) |
| | *α* | [°] | 81.465(8) | 90 | 98.452(4) | 90 |
| | *β* | [°] | 83.577(8) | 94.275(3) | 96.523(3) | 97.710(5) |
| | *γ* | [°] | 77.620(8) | 90 | 98.888(3) | 90 |
| Unit lattice volume | *V* | [Å³] | 991.32(17) | 4494.7(3) | 3378.3(2) | 1924.01(16) |
| Number of structural units in unit lattice | *Z* | [-] | 2 | 4 | 2 | 4 |
| R factor | | | 0.0543 | 0.1310 | 0.1125 | 0.1305 |
| R_{ω} factor | | | 0.1449 | 0.3533 | 0.2136 | 0.3397 |

| | | | | | | |
|---|---|---|---|---|---|---|
| *Analysis including DMF (3 molecules) as solvent molecule. | | | | | | |

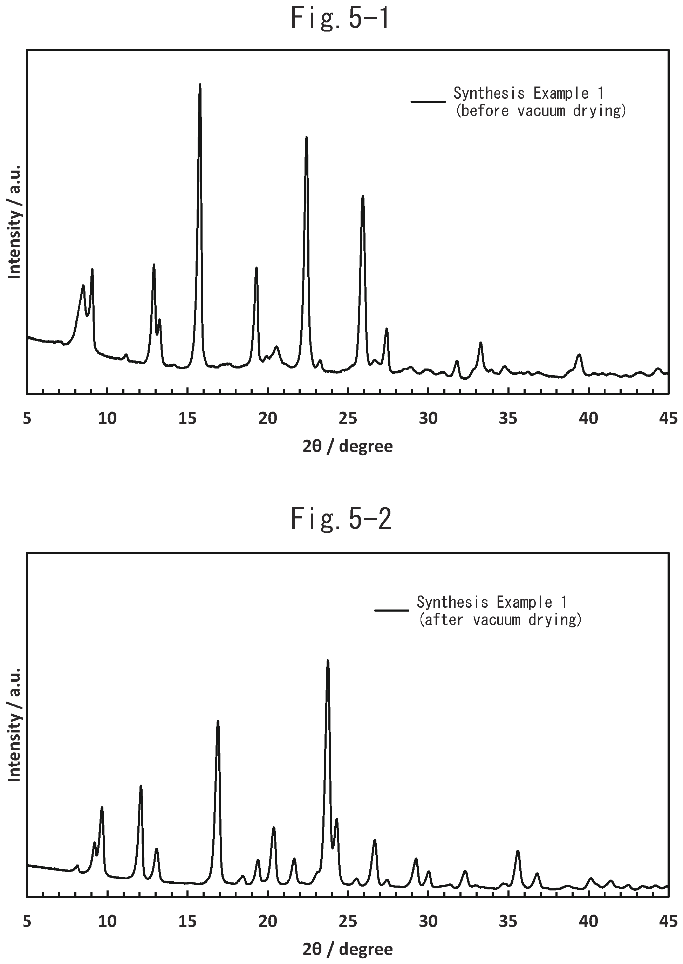

The precipitated metal complex was recovered from the reaction solution, from which the crystals for the single crystal X-ray structure analysis had been taken out, by suction filtration, and then washed with methanol three times. Next, vacuum drying (10 to 20 Pa) was performed at 200°C for 2 hours using a vacuum dryer DRV320DA (ADVANTEC TOYO KAISHA, LTD.) to obtain 1.49 g of the desired metal complex A (yield 87%). The powder X-ray diffraction pattern of the obtained metal complex A before vacuum drying and the powder X-ray diffraction pattern thereof after vacuum drying are illustrated in Figs. 5-1 and 5-2, respectively.

Since the metal complex A has a CID structure before being subjected to vacuum drying as illustrated in Figs. 3 and 4, Fig. 5-1 illustrates the powder X-ray diffraction pattern peculiar to the CID structure. Furthermore, since Figs. 5-1 and 5-2 illustrate approximately the same diffraction patterns, it can be seen that the CID structure is maintained when the solvent is removed as well. The change in diffraction pattern before the vacuum drying and after the vacuum drying occurs since the distance between the sheets is narrowed while the CID structure is maintained by the removal of solvent. In the CID structure before vacuum drying, relatively strong peaks are observed at diffraction angles (2θ) of 8.5°, 9.5°, 12.9°, 15.8°, 19.3°, 22.4°, and 26.0°, for example. In the CID structure after vacuum drying, relatively strong peaks are observed at diffraction angles (2θ) of 9.2°, 9.7°, 12.1°, 13.1°, 16.9°, 20.4°, 23.7°, and 26.7°, for example.

### <Synthesis Example 2> Synthesis of Metal Complex B

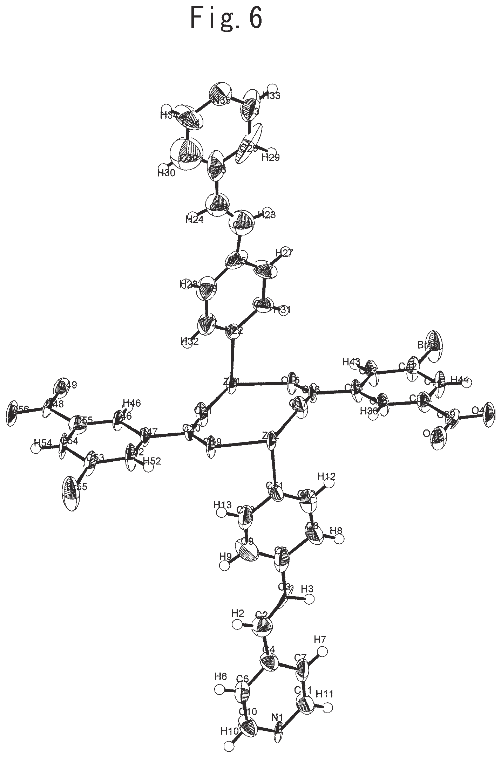

A metal complex B was obtained by 1.72 g (yield 87%) in the same manner as in Synthesis Example 1 except that 4-hydroxyisophthalic acid was replaced with 0.980 g (4.0 mmol) of 5-bromoisophthalic acid. A part of the precipitated crystals were taken out from the reaction solution, and the single crystal X-ray structure analysis was carried out on this crystal. Fig. 6 illustrates the ORTEP diagram acquired by the single crystal X-ray structure analysis. As a result of the structure analysis, it was found that the metal complex B had the composition of zinc dication : dianion of 5-bromoisophthalic acid : trans-1,2-bis(4-pyridyl)ethylene = 1 : 1 : 1. Table 1 presents the lattice constant and the like.

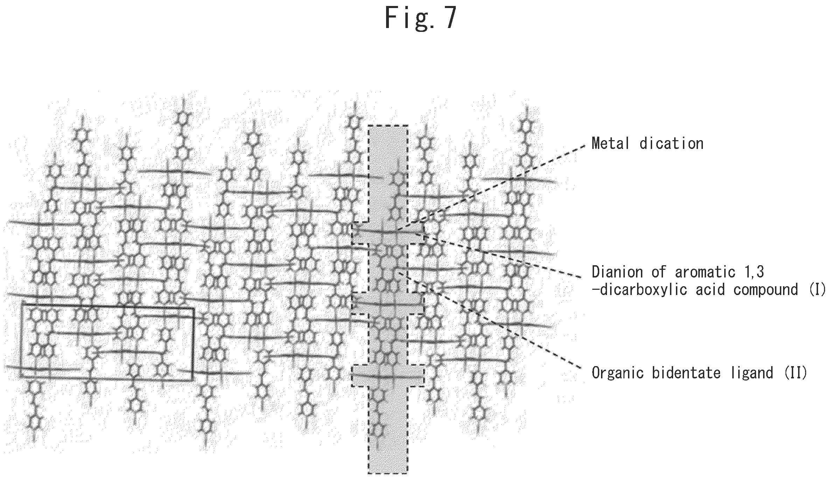

Fig. 7 illustrates the crystal structure model of the metal complex B. It can be seen from Fig. 7 that the crystal of the metal complex B has a structure (CID structure) in which two-dimensional sheets are laminated while being interdigitated with each other.

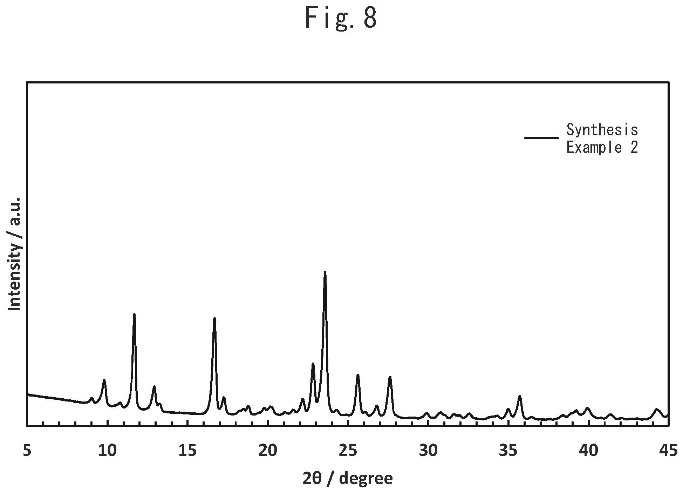

Fig. 8 illustrates the powder X-ray diffraction pattern of the obtained metal complex B after vacuum drying. Relatively strong peaks are observed at diffraction angles (2θ) of 9.1°, 9.9°, 11.7°, 13.0°, 16.7°, 23.6°, and 25.7°, and the diffraction pattern is well consistent with the diffraction pattern peculiar to the CID structure illustrated in Fig. 5-2.

### <Synthesis Example 3> Synthesis of Metal Complex C

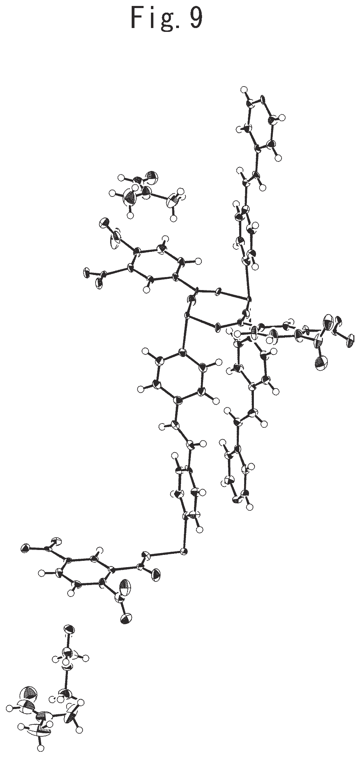

A solution of 0.149 g (0.5 mmol) of zinc nitrate hexahydrate dissolved in 10 mL of N,N-dimethylformamide was added to a solution of 0.106 g (0.5 mmol) of 4-nitroisophthalic acid and 0.091 g (0.5 mmol) of trans-1,2-bis(4-pyridyl)ethylene dissolved in 10 mL of N,N-dimethylformamide, and then the mixture was left to still stand at normal pressure and 120°C for 48 hours. A part of the precipitated crystals was taken out and subjected to single crystal X-ray structure analysis. The ORTEP diagram acquired by the single crystal X-ray structure analysis is illustrated in Fig. 9. The crystals contained N,N-dimethylformamide, which was a solvent molecule, and the analysis was performed including this molecule. As a result of the structure analysis, it was found that the metal complex C had the composition of zinc dication : dianion of 4-nitroisophthalic acid : trans-1,2-bis(4-pyridyl)ethylene = 1 : 1 : 1. Table 1 presents the lattice constant and the like.

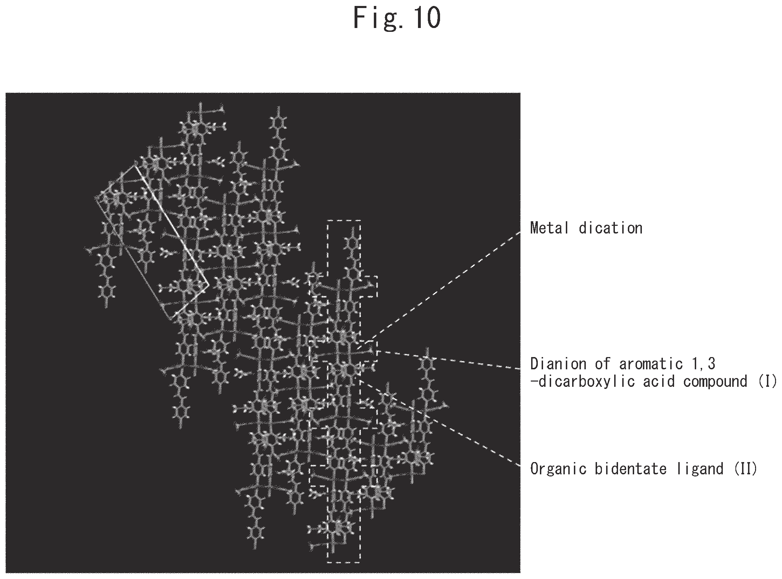

Fig. 10 illustrates the crystal structure model of the metal complex C. It can be seen from Fig. 10 that the crystal of the metal complex C has a structure (CID structure) in which two-dimensional sheets are laminated while being interdigitated with each other.

### <Synthesis Example 4> Synthesis of Metal Complex D

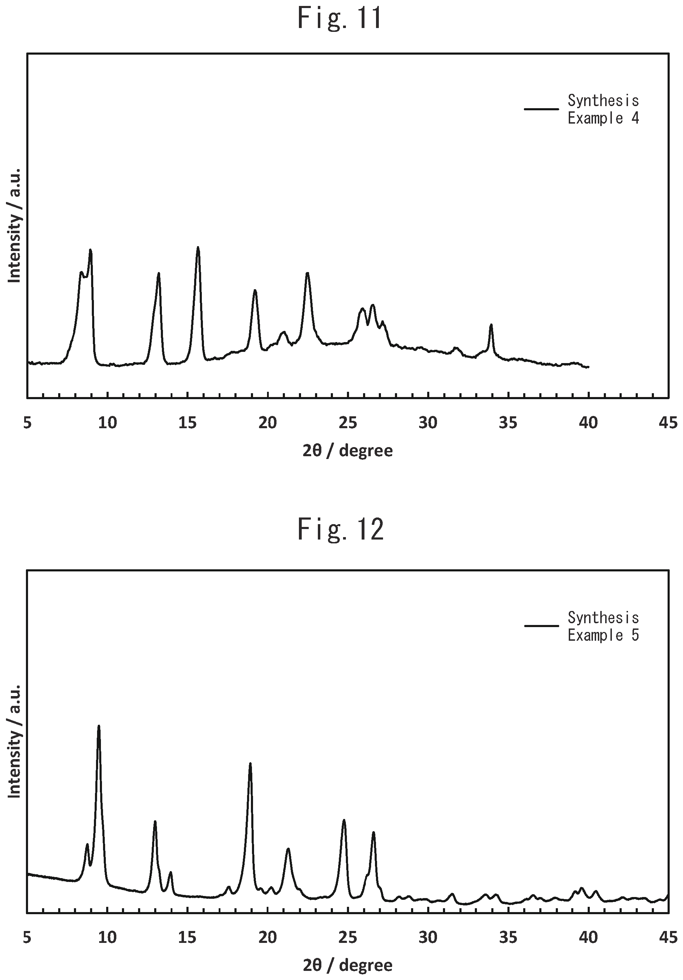

In 12 mL of N,N-dimethylformamide and 10 mL of water, 0.097 g (0.4 mmol) of copper nitrate trihydrate, 0.073 g (0.4 mmol) of 4-hydroxyisophthalic acid, and 0.073 g (0.4 mmol) of trans-1,2-bis(4-pyridyl)ethylene were dissolved and left to still stand at normal pressure and 95°C for 16 hours. A part of the precipitated crystals was taken out and subjected to powder X-ray structure analysis. The result is illustrated in Fig. 11. Relatively strong peaks are observed at diffraction angles (2θ) of 8.4°, 8.9°, 13.3°, 15.7°, 19.3°, and 22.6°, and the diffraction pattern is well consistent with the diffraction pattern peculiar to the CID structure illustrated in Fig. 5-1. It can thus be seen that the metal complex D obtained in Synthesis Example 4 has the composition of copper dication : dianion of 4-hydroxyisophthalic acid : trans-1,2-bis(4-pyridyl)ethylene = 1 : 1 : 1, and has a structure (CID structure) in which two-dimensional sheets are laminated while being interdigitated with each other.

### <Synthesis Example 5> Synthesis of Metal Complex E

In 60 mL of N,N-dimethylformamide and 50 mL of water, 0.595 g (2.0 mmol) of zinc nitrate hexahydrate, 0.310 g (2.0 mmol) of 1H-2,5-pyrroledicarboxylic acid, and 0.369 g (2.0 mmol) of trans-1,2-bis(4-pyridyl)ethylene were dissolved, and the mixture was stirred at normal pressure and 80°C for 16 hours. The precipitated metal complex was recovered by suction filtration and then washed with methanol three times. Next, vacuum drying (10 to 20 Pa) was performed at 200°C for 2 hours using a vacuum dryer DRV320DA (ADVANTEC TOYO KAISHA, LTD.) to obtain the desired metal complex E: 0.396 g (yield 50%). The powder X-ray diffraction pattern of the obtained metal complex E is illustrated in Fig. 12. Relatively strong peaks are observed at diffraction angles (2θ) of 8.8°, 9.4°, 13.1°, 14.0°, 19.0°, 21.4°, 24.9°, and 26.7°, and the diffraction pattern is well consistent with the diffraction pattern peculiar to the CID structure illustrated in Fig. 5-2. It can thus be seen that the metal complex E obtained in Synthesis Example 5 has the composition of zinc dication : dianion of 1H-2,5-pyrroledicarboxylic acid : trans-1,2-bis(4-pyridyl)ethylene = 1 : 1 : 1, and has a structure (CID structure) in which two-dimensional sheets are laminated while being interdigitated with each other.

### <Synthesis Example 6> Synthesis of Metal Complex F

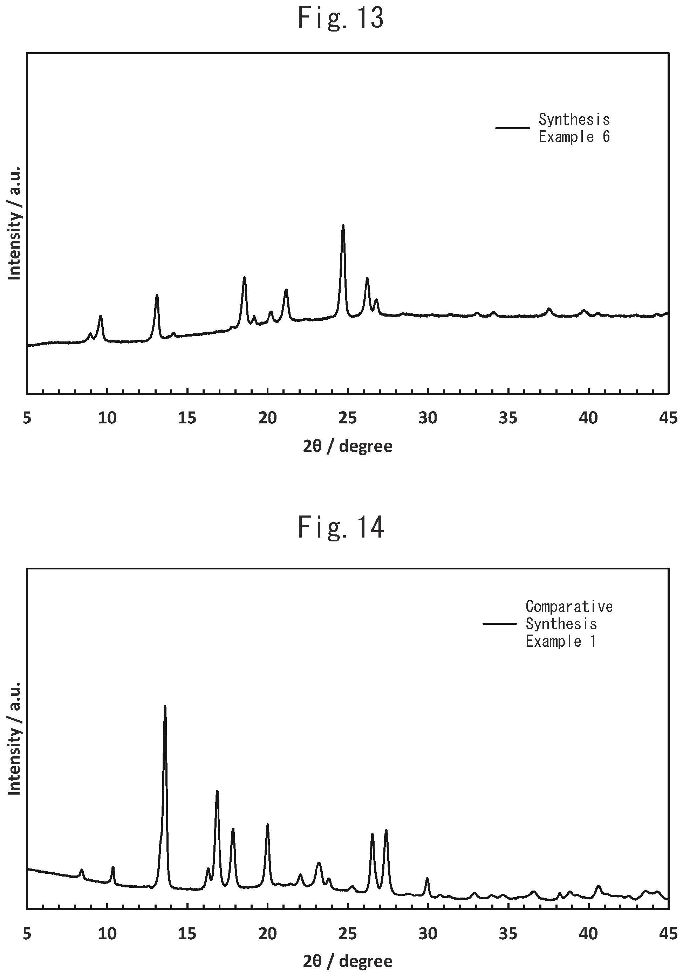

A desired metal complex F: 0.5037 g (yield 63%) was obtained in the same manner as in Synthesis Example 5 except that zinc nitrate hexahydrate was replaced with 0.5877 g (2.0 mmol) of cobalt nitrate hexahydrate and the reaction temperature was set to 95°C. The powder X-ray diffraction pattern of the obtained metal complex F is illustrated in Fig. 13. Relatively strong peaks are observed at diffraction angles (2θ) of 9.0°, 9.6°, 13.2°, 18.6°, 21.2°, 24.7°, and 26.2°, and the diffraction pattern is well consistent with the diffraction pattern peculiar to the CID structure illustrated in Fig. 5-2. It can thus be seen that the metal complex F obtained in Synthesis Example 6 has the composition of cobalt dication : dianion of 1H-2,5-pyrroledicarboxylic acid : trans-1,2-bis(4-pyridyl)ethylene = 1 : 1 : 1, and has a structure (CID structure) in which two-dimensional sheets are laminated while being interdigitated with each other.

### <Comparative Synthesis Example 1> Synthesis of Comparative Metal Complex P

A comparative metal complex P was synthesized by stirring zinc nitrate hexahydrate, isophthalic acid and trans-1,2-bis(4-pyridyl)ethylene in a mixed solution of N,N-dimethylformamide and water at normal pressure and 80°C for 72 hours in conformity with the method described in Example 1 of Japanese Patent Application Publication No. 2013-107826. The results of the single crystal X-ray structure analysis for the comparative metal complex P are described in Japanese Patent Application Publication No. 2013-107826, and the comparative metal complex P has a structure in which pseudo-diamond skeletons are triply interpenetrated.

The powder X-ray diffraction pattern of the comparative metal complex P is illustrated in Fig. 14. Relatively strong peaks are observed at diffraction angles (2θ) of 13.6°, 16.9°, 17.9°, 20.0°, 26.6°, and 27.5°.

### <Comparative Synthesis Example 2> Synthesis of Comparative Metal Complex Q

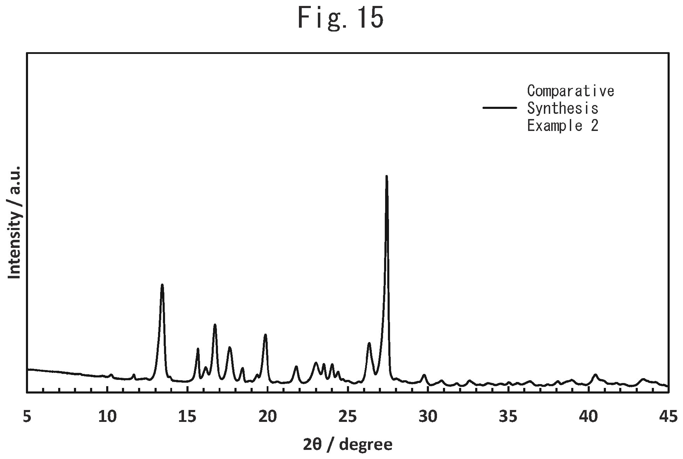

A comparative metal complex Q: 0.394 g was obtained by conducting the same reaction as in Synthesis Example 1 except that the solvent was replaced with only 200 mL of water and the time was set to 18 hours. The powder X-ray diffraction pattern of the obtained comparative metal complex Q is illustrated in Fig. 15. Relatively strong peaks are observed at diffraction angles (2θ) of 13.5°, 16.8°, 17.8°, 19.9°, 26.4°, and 27.5°, and the diffraction pattern is well consistent with the diffraction pattern peculiar to the structure, in which pseudo-diamond skeletons are triply interpenetrated, illustrated in Fig. 14. Therefore, it can be seen that the comparative metal complex Q obtained in Comparative Synthesis Example 2 has a structure, in which pseudo-diamond skeletons are triply interpenetrated.

### <Comparative Synthesis Example 3> Synthesis of Comparative Metal Complex R

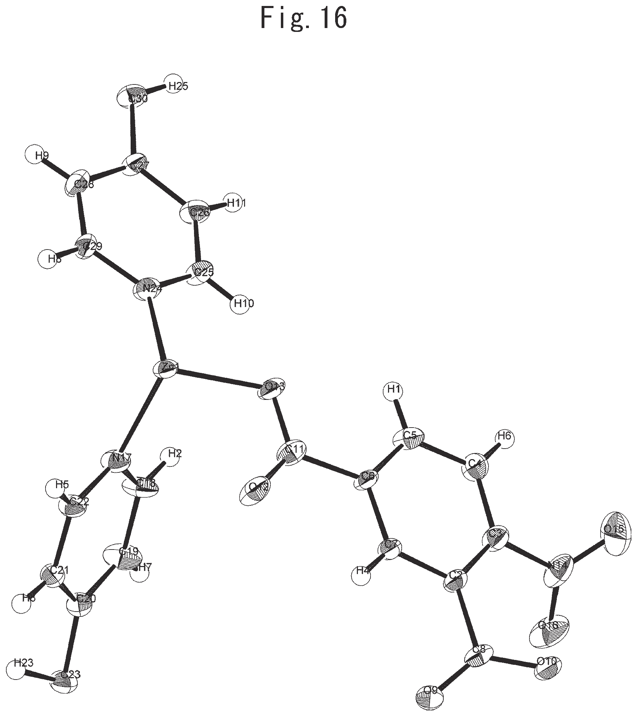

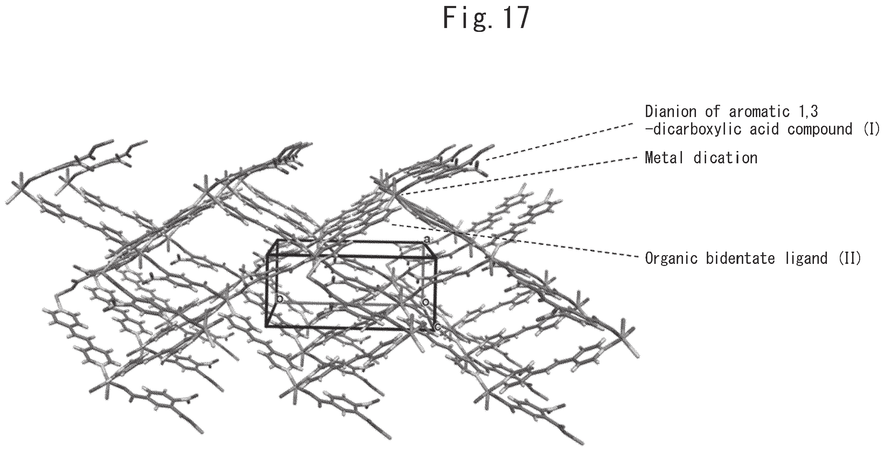

In 12 mL of N,N-dimethylformamide and 10 mL of water, 0.119 g (0.4 mmol) of zinc nitrate hexahydrate, 0.084 g (0.4 mmol) of 4-nitroisophthalic acid, and 0.073 g (0.4 mmol) of trans-1,2-bis(4-pyridyl)ethylene were dissolved and left to still stand at 95°C for 16 hours. A part of the precipitated crystals was taken out and subjected to single crystal X-ray structure analysis. Fig. 16 illustrates the ORTEP diagram acquired by the single crystal X-ray structure analysis. Fig. 17 illustrates the crystal structure model of a comparative metal complex R. It can be seen from Fig. 17 that the comparative metal complex R has a structure, in which pseudo-diamond skeletons are triply interpenetrated. Table 1 presents the lattice constant and the like.

### <Comparative Synthesis Example 4> Synthesis of Comparative Metal Complex S

In 12 mL of N,N-dimethylformamide and 10 mL of water, 0.119 g (0.4 mmol) of zinc nitrate hexahydrate, 0.073 g (0.4 mmol) of 4-hydroxyisophthalic acid, and 0.074 g (0.4 mmol) of 1,2-bis(4-pyridyl)ethane were dissolved and left to still stand at 95°C for 16 hours to obtain a comparative metal complex S as a precipitate.

The reaction conditions and results of the above-mentioned Synthesis Examples are presented in Table 2.

### [Table 2]

**Table 2**

| | | Aromatic 1,3-dicarboxylic acid compound (I) | Metal dication | Organic bidentate ligand (II) | Reaction conditions | | | | Structure of metal complex |
|---|---|---|---|---|---|---|---|---|---|
| | | | | | Solvent | Volume mixing ratio | Temperature [°C] | Time [h] | |
| Synthesis Example 1 | Metal complex A | 4-Hydroxyisophthalic acid | Zn²⁺ | 1,2-Bis(4-pyridyl)ethylene | H₂O-DMF | 0.83 | 95 | 16 | CID structure |
| Synthesis Example 2 | Metal complex B | 5-Bromoisophthalic acid | Zn²⁺ | 1,2-Bis(4-pyridyl)ethylene | H₂O-DMF | 0.83 | 95 | 16 | CID structure |
| Synthesis Example 3 | Metal complex C | 4-Nitroisophthalic acid | Zn²⁺ | 1,2-Bis(4-pyridyl)ethylene | DMF | 0 | 120 | 48 | CID structure |
| Synthesis Example 4 | Metal complex D | 4-Hydroxyisophthalic acid | Cu²⁺ | 1,2-Bis(4-pyridyl)ethylene | H₂O-DMF | 0.83 | 95 | 16 | CID structure |
| Synthesis Example 5 | Metal complex E | 1H-2,5-pyrroledicarboxylic acid | Zn²⁺ | 1,2-Bis(4-pyridyl)ethylene | H₂O-DMF | 0.83 | 80 | 16 | CID structure |
| Synthesis Example 6 | Metal complex F | 1H-2,5-pyrroledicarboxylic acid | Co²⁺ | 1,2-Bis(4-pyridyl)ethylene | H₂O-DMF | 0.83 | 95 | 16 | CID structure |
| Comparative Synthesis Example 1 | Comparative metal complex P | Isophthalic acid | Zn²⁺ | 1,2-Bis(4-pyridyl)ethylene | H₂O-DMF | 0.83 | 80 | 72 | Pseudo-diamond structure |
| Comparative Synthesis Example 2 | Comparative metal complex Q | 4-Hydroxyisophthalic acid | Zn²⁺ | 1,2-Bis(4-pyrldyl)ethylene | H₂O | - | 95 | 18 | Pseudo-diamond structure |
| Comparative Synthesis Example 3 | Comparative metal complex R | 4-Nitroisophthalic acid | Zn²⁺ | 1,2-Bis(4-pyridyl)ethylene | H₂O-DMF | 0.83 | 95 | 16 | Pseudo-diamond structure |
| Comparative Synthesis Example 4 | Comparative metal complex S | 4-Hydroxyisophthalic acid | Zn²⁺ | 1,2-Bis(4-pyridyl)ethane | H₂O-DMF | 0.83 | 95 | 16 | - |

| | | | | | | | | | |
|---|---|---|---|---|---|---|---|---|---|
| Volume mixing ratio: Volume of H₂O (mL)/volume of N,N-dimethylformamide (mL) CID structure: Structure in which two-dimensional sheets are laminated while being interdigitated with each other. | | | | | | | | | |

### <Example 1>

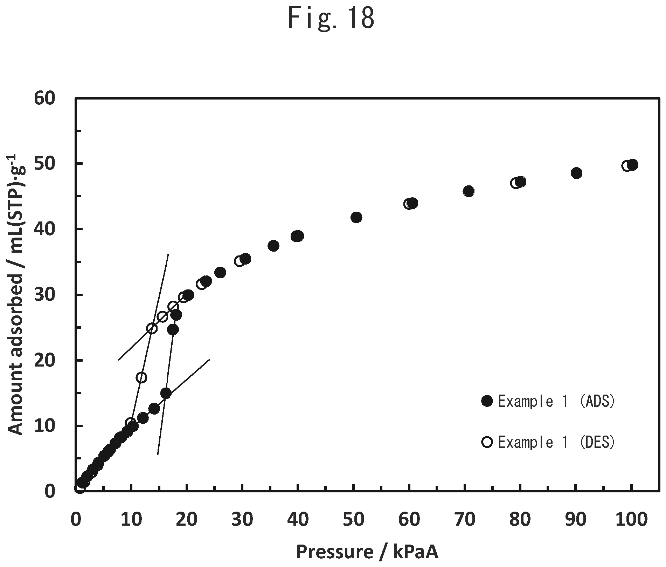

The adsorption isotherm (ADS) and desorption isotherm (DES) of carbon dioxide at 298 K were measured for the metal complex A obtained in Synthesis Example 1. The result is illustrated in Fig. 18. From the adsorption and desorption isotherms, the gate opening pressure was 16 kPaA and the gate closing pressure was 14 kPaA.

### <Example 2>

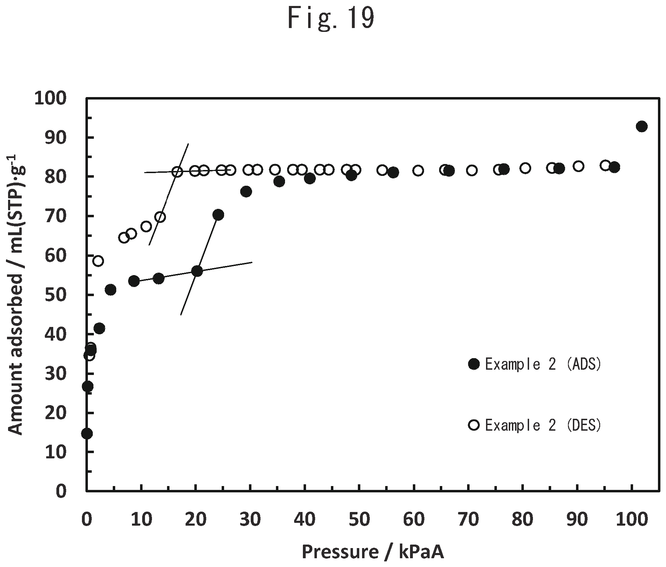

The adsorption isotherm (ADS) and desorption isotherm (DES) of carbon dioxide at 195 K were measured for the metal complex B obtained in Synthesis Example 2. The result is illustrated in Fig. 19. From the adsorption and desorption isotherms, the gate opening pressure was 20 kPaA and the gate closing pressure was 17 kPaA.

### <Example 3>

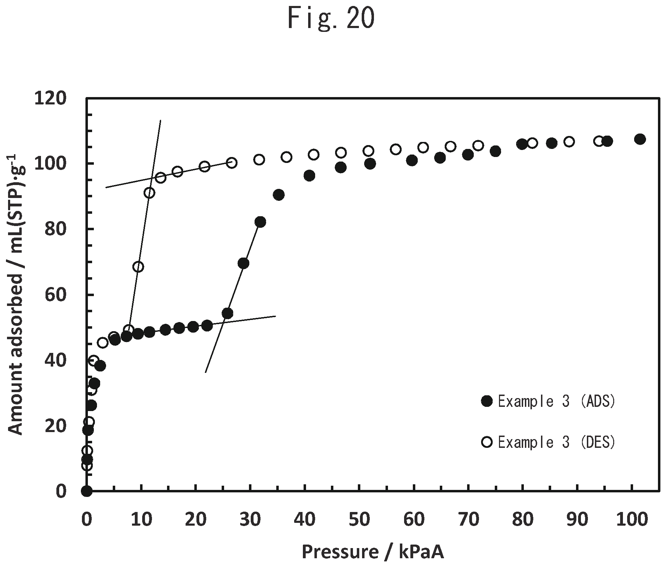

The adsorption isotherm (ADS) and desorption isotherm (DES) of carbon dioxide at 195 K were measured for the metal complex C obtained in Synthesis Example 3. The result is illustrated in Fig. 20. From the adsorption and desorption isotherms, the gate opening pressure was 25 kPaA and the gate closing pressure was 12 kPaA.

### <Example 4>

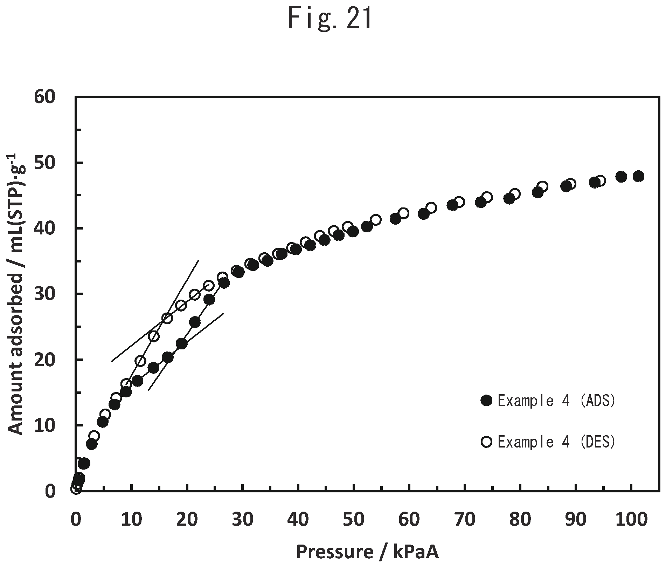

The adsorption isotherm (ADS) and desorption isotherm (DES) of carbon dioxide at 298 K were measured for the metal complex D obtained in Synthesis Example 4. The result is illustrated in Fig. 21. From the adsorption and desorption isotherms, the gate opening pressure was 18 kPaA and the gate closing pressure was 16 kPaA.

### <Example 5>

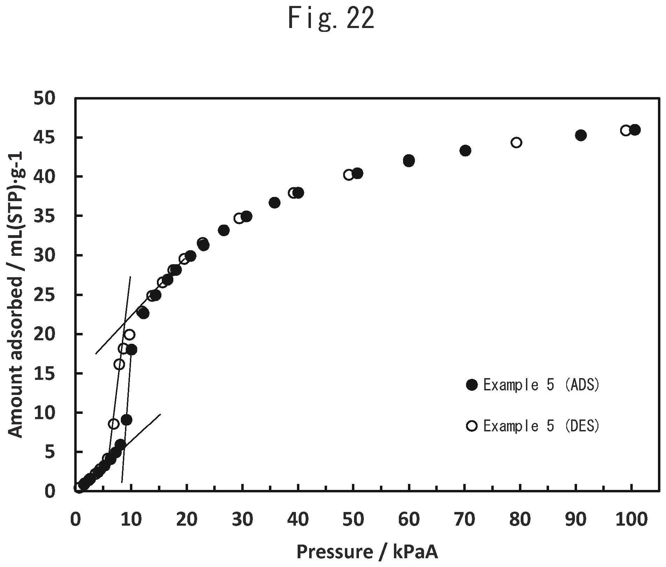

The adsorption isotherm (ADS) and desorption isotherm (DES) of carbon dioxide at 298 K were measured for the metal complex E obtained in Synthesis Example 5. The result is illustrated in Fig. 22. From the adsorption and desorption isotherms, the gate opening pressure was 9 kPaA and the gate closing pressure was 9 kPaA.

### <Example 6>

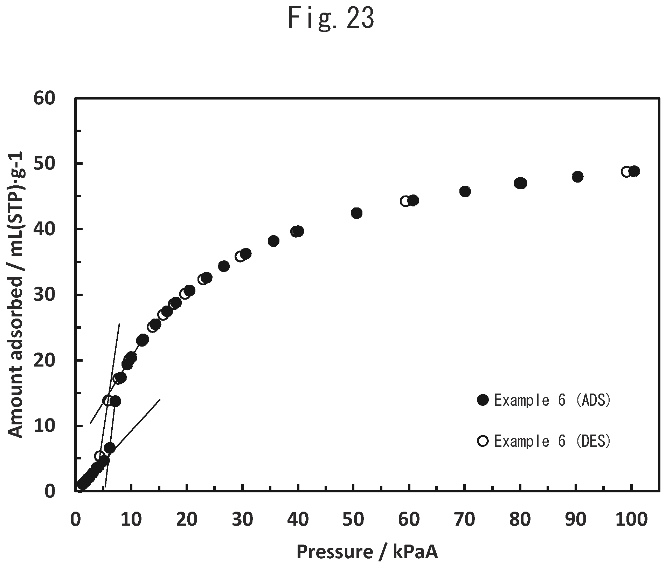

The adsorption isotherm (ADS) and desorption isotherm (DES) of carbon dioxide at 298 K were measured for the metal complex F obtained in Synthesis Example 6. The result is illustrated in Fig. 23. From the adsorption and desorption isotherms, the gate opening pressure was 6 kPaA and the gate closing pressure was 6 kPaA.

### <Comparative Example 1>

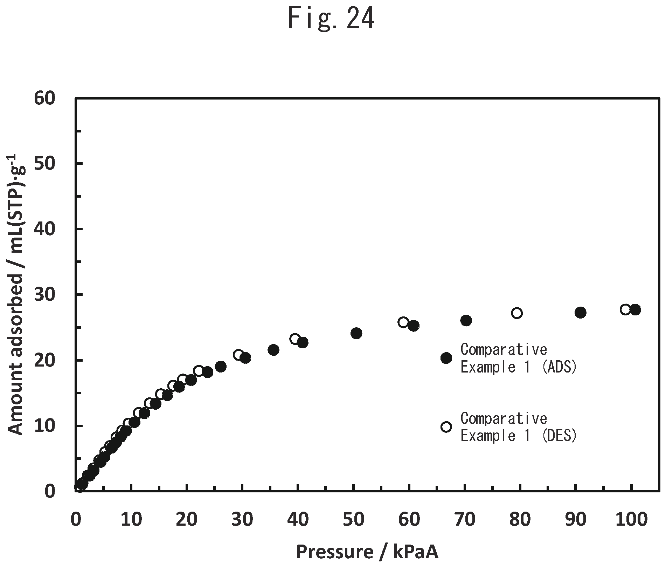

The adsorption isotherm (ADS) and desorption isotherm (DES) of carbon dioxide at 298 K were measured for the comparative metal complex Q obtained in Comparative Synthesis Example 2. The result is illustrated in Fig. 24. The comparative metal complex Q, unlike in Example 1, does not exhibit a gating phenomenon. Such a metal complex is not preferable since the desorption step must be carried out at an extremely low pressure in the separation and recovery of carbon dioxide by the PSA method and the cost required for the recovery increases.

### <Comparative Example 2>

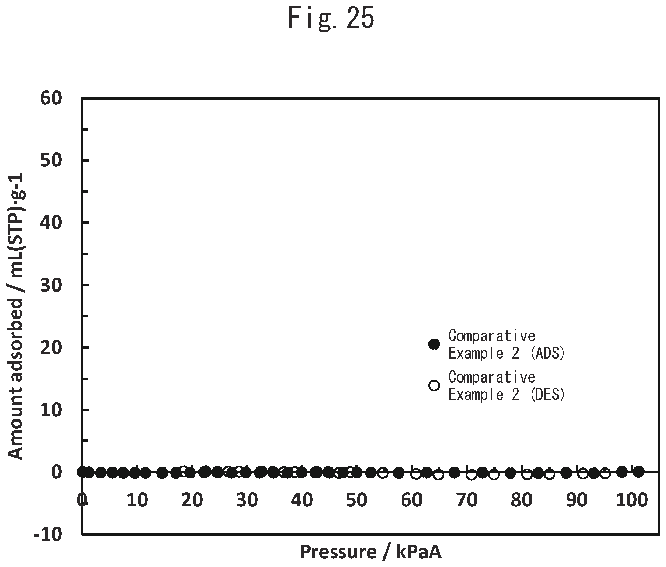

The adsorption isotherm (ADS) and desorption isotherm (DES) of carbon dioxide at 298 K were measured for the comparative metal complex R obtained in Comparative Synthesis Example 3, but carbon dioxide adsorption was not observed. The result is illustrated in Fig. 25.

### <Comparative Example 3>

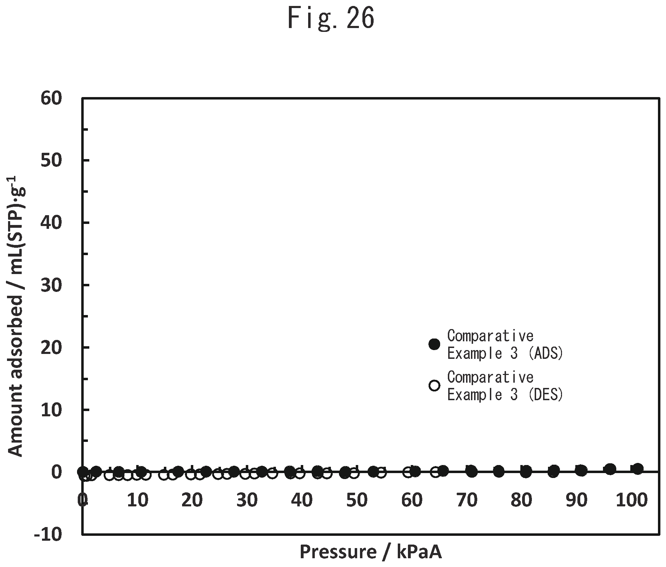

The adsorption isotherm (ADS) and desorption isotherm (DES) of carbon dioxide at 195 K were measured for the comparative metal complex S obtained in Comparative Synthesis Example 4, but carbon dioxide adsorption was not observed. The result is illustrated in Fig. 26.

The gate opening pressure and gate closing pressure of Examples 1 to 6 and Comparative Examples 1 to 3 are presented in Table 3. From Examples 1 to 6, the metal complexes A to F have low gate opening pressures and low gate closing pressures, and thus are useful as a separating agent that can separate and recover carbon dioxide from a mixed gas containing carbon dioxide at a low concentration with a high energy efficiency.

### [Table 3]

**Table 3**

| | | Aromatic 1,3-dicarboxylic acid compound (I) | Metal dication | Organic bidentate ligand (II) | Adsorution and desorption isotherms | | |
|---|---|---|---|---|---|---|---|
| | | | | | Measured temperature [K] | Gate opening pressure [kPaA] | Gate closing pressure [kPaA] |
| Example 1 | Metal complex A | 4-Hydroxyisophthalic acid | Zn²⁺ | 1,2-Bis(4-pyridyl)ethylene | 298 | 16 | 14 |
| Example 2 | Metal complex B | 5-Bromoisophthalic acid | Zn²⁺ | 1,2-Bis(4-pyridyl)ethylene | 195 | 20 | 17 |
| Example 3 | Metal complex C | 4-Nitroisophthalic acid | Zn²⁺ | 1,2-Bis(4-pyridyl)ethylene | 195 | 25 | 12 |
| Example 4 | Metal complex D | 4-Hydroxyisophthalic acid | Cu²⁺ | 1,2-Bis(4-pyridyl)ethylene | 298 | 18 | 16 |
| Example 5 | Metal complex E | 1H-2,5-pyrroledicarboxylic acid | Zn²⁺ | 1,2-Bis(4-pyridyl)ethylene | 298 | 9 | 9 |
| Example 6 | Metal complex F | 1H-2,5-pyrroledicarboxylic acid | Co²⁺ | 1,2-Bis(4-pyridyl)ethylene | 298 | 6 | 6 |
| Comparative Example 1 | Comparative metal complex Q | 4-Hydroxyisophthalic acid | Zn²⁺ | 1,2-Bis(4-pyridyl)ethylene | 298 | Gating phenomenon is not observed | |
| Comparative Example 2 | Comparative metal complex R | 4-Nitroisophthalic acid | Zn²⁺ | 1,2-Bis(4-pyridyl)ethylene | 298 | CO₂ adsorption is not observed | |
| Comparative Example 3 | Comparative metal complex S | 4-Hydroxyisophthalic acid | Zn²⁺ | 1,2-Bis(4-pyridyl)ethane | 195 | CO₂ adsorption is not observed | |

### <Example 7> Separation of Mixed Gas (Carbon Dioxide and Nitrogen)

As a separating agent, the metal complex A (10.6 g) obtained in Synthesis Example 1 was filled in a jacketed stainless tube having a volume of 32 mL (internal diameter 1 cm, length 40 cm). The adsorption and desorption operation of a mixed gas of carbon dioxide (CO₂) : nitrogen (N₂) = 1 : 9 (volume ratio) was repeated while water at a constant temperature of 25°C was circulated through the jacket. In the adsorption operation, the mixed gas was supplied to the stainless tube at a pressure 0.3 MPaA (CO₂ partial pressure of 30 kPaA). In the desorption operation, the interior of the stainless tube was degassed using a diaphragm pump to recover the desorbed gas. The adsorption operation and desorption operation described above were repeated, the desorbed gas in a state (steady state) in which the carbon dioxide concentration in the gas to be desorbed reached a constant value was analyzed, and the CO₂ concentration was found to be 58.9% by volume. In other words, the CO₂ concentration was concentrated by approximately six times from 10% by volume.

From the results of Example 7, it has been demonstrated that the metal complex A is excellent as a separating agent for separating carbon dioxide from a mixed gas containing carbon dioxide at a low concentration.

## Claims

1. A metal complex comprising:
a dianion of an aromatic 1,3-dicarboxylic acid compound (I);
a metal dication that is a dication of a metal selected from magnesium, calcium, chromium, molybdenum, tungsten, manganese, iron, ruthenium, cobalt, rhodium, nickel, palladium, copper, zinc, and cadmium; and
an organic bidentate ligand (II) capable of coordinating to the metal dication, represented by general formula (1) below: 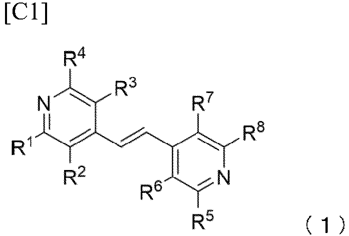 wherein R¹, R², R³, R⁴, R⁵, R⁶, R⁷, and R⁸ are each independently a hydrogen atom, an alkyl group having 1 to 4 carbon atoms, an alkoxy group having 1 to 4 carbon atoms, a formyl group, an acyloxy group, an alkoxycarbonyl group having 2 to 4 carbon atoms, a nitro group, a cyano group, an amino group, a monoalkylamino group having 1 to 4 carbon atoms, a dialkylamino group having 2 to 4 carbon atoms, an acylamino group having 2 to 4 carbon atoms, or a halogen atom,
the metal complex having a structure in which two-dimensional sheets are laminated while being interdigitated with each other.

2. The metal complex according to claim 1, wherein a gate opening pressure and a gate closing pressure are both 1 to 60 kPaA in adsorption and desorption isotherms of carbon dioxide at any measured temperature of 150 to 350 K.

3. The metal complex according to claim 1 or 2, wherein the aromatic 1,3-dicarboxylic acid compound (I) is a monocyclic 6-membered aromatic 1,3-dicarboxylic acid compound or a monocyclic 5-membered aromatic 1,3-dicarboxylic acid compound.

4. The metal complex according to claim 1 or 2, wherein the aromatic 1,3-dicarboxylic acid compound (I) is a monocyclic 6-membered aromatic 1,3-dicarboxylic acid compound, wherein a ring exhibiting aromaticity in the monocyclic 6-membered aromatic 1,3-dicarboxylic acid compound has at least one substituent selected from a halogen atom, a hydroxy group, and a nitro group in addition to two carboxy groups.

5. The metal complex according to claim 4, wherein the aromatic 1,3-dicarboxylic acid compound (I) is represented by general formula (2) below: 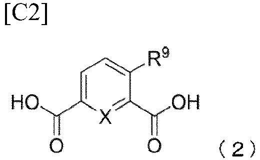 wherein X is CH or N and R⁹ is a halogen atom, a hydroxy group, or a nitro group.

6. The metal complex according to claim 1 or 2, wherein the aromatic 1,3-dicarboxylic acid compound (I) is represented by general formula (3) below: 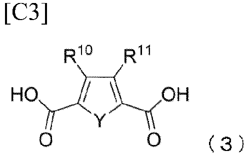 wherein Y is NH, S, or O, R¹⁰ and R¹¹ are each independently a hydrogen atom or an alkoxy group having 1 to 3 carbon atoms, and R¹⁰ and R¹¹ may be bonded to each other to form a ring structure.

7. The metal complex according to claim 6, wherein both R¹⁰ and R¹¹ are hydrogen atoms.

8. The metal complex according to claim 1 or 2, wherein a composition ratio (molar ratio) of the metal dication, the dianion of the aromatic 1,3-dicarboxylic acid compound (I), and the organic bidentate ligand (II) in the metal complex is 1 : 0.9 to 1.1 : 0.9 to 1.1.

9. A separating agent comprising the metal complex according to claim 1 or 2.

10. A method for separating a target gas, the method comprising:
bringing a mixed gas comprising a target gas that is to be separated and other gases into contact with the separating agent according to claim 9, and adsorbing the target gas onto the separating agent; and
desorbing the target gas.

11. The method for separating a target gas according to claim 10, wherein the mixed gas is a gas that comprises at least two kinds selected from an oxygen gas, a nitrogen gas, water vapor, a nitrogen oxide gas, a sulfur oxide gas, a hydrogen gas, a carbon monoxide gas, a carbon dioxide gas, a hydrogen sulfide gas, ammonia, a hydrocarbon gas having 1 to 4 carbon atoms, a noble gas, and siloxane.

12. The method for separating a target gas according to claim 10, wherein the target gas is a carbon dioxide gas.

13. The method for separating a target gas according to claim 10, wherein the mixed gas is a combustion exhaust gas emitted from a plant and the target gas is a carbon dioxide gas.

14. A method for separating a target gas, the method comprising:
bringing a mixed gas comprising a target gas that is to be separated and other gases into contact with a separating agent, and adsorbing the target gas onto the separating agent; and
desorbing the target gas,
in which the separating agent comprises a metal complex having a structure in which two-dimensional sheets are laminated while being interdigitated with each other, in which a gate opening pressure and a gate closing pressure are both 1 to 60 kPaA in an adsorption isotherm of carbon dioxide at an operating temperature.

15. The method for separating a target gas according to claim 14, wherein the mixed gas is a gas that comprises at least two kinds selected from an oxygen gas, a nitrogen gas, water vapor, a nitrogen oxide gas, a sulfur oxide gas, a hydrogen gas, a carbon monoxide gas, a carbon dioxide gas, a hydrogen sulfide gas, ammonia, a hydrocarbon gas having 1 to 4 carbon atoms, a noble gas, or siloxane.

16. The method for separating a target gas according to claim 14 or 15, wherein the target gas is a carbon dioxide gas.

17. The method for separating a target gas according to claim 14, wherein the metal complex is the metal complex according to claim 1 or 2.

18. The method for separating a target gas according to claim 16, wherein a partial pressure of carbon dioxide in the mixed gas is 1 to 30 kPaA.

19. The method for separating a target gas according to claim 14 or 15, wherein the operating temperature is 150 to 350 K.

20. A carbon dioxide separation apparatus comprising a separating agent comprising the metal complex according to claim 1 or 2.

21. A metal complex having a structure in which two-dimensional sheets are laminated while being interdigitated with each other, wherein a gate opening pressure and a gate closing pressure are both 1 to 60 kPaA in adsorption and desorption isotherms of carbon dioxide at any measured temperature of 150 to 350 K.
